# EUROPEAN PATENT APPLICATION

(11) **EP 4 775 675 A1**
(43) Date of publication of application: **15.07.2026**
(21) Application number: 24861927.2
(22) Date of filing: 02.09.2024
(51) Int. Cl.: C12N 15/09, C07K 16/28, C12N 1/15, C12N 1/19, C12N 1/21, C12N 5/10, C12P 21/08

(54) **ANTI-BDCA-2 SPECIFIC ANTIBODY AND USE THEREOF**

(30) Priority: 05.09.2023 CN 202311136732
(71) Applicant: Shanghai Hongcheng Biopharmaceutical Co., Ltd., Shanghai 201315 (CN)
(72) Inventor: MENG, Xiangmin, Shanghai 201203 (CN); MIN, Hao, Shanghai 201203 (CN); WANG, Yonghui, Shanghai 201203 (CN); TAO, Yao, Shanghai 201203 (CN); WANG, Xingding, Shanghai 201203 (CN); SHENG, Hanzhe, Shanghai 201203 (CN); WANG, Ning, Shanghai 201203 (CN); JIANG, Diandong, Shanghai 201203 (CN)
(74) Representative: Vossius & Partner Patentanwälte Rechtsanwälte mbB
(86) International application number: PCT/CN2024/116265
(87) International publication number: WO 2025/051083

(57) **Abstract**

The present disclosure relates to an anti-BDCA-2 antibody or antigen-binding fragment thereof. The present disclosure also relates to compositions comprising the anti-BDCA-2 antibody or antigen-binding fragment thereof, nucleic acids encoding the anti-BDCA-2 antibody or antigen-binding fragment thereof, vectors and host cells comprising the nucleic acids. The present disclosure further relates to therapeutic uses of the anti-BDCA-2 antibodies or antigen-binding fragments thereof and pharmaceutical compositions comprising the same.

## Description

### TECHNICAL FIELD

The present disclosure relates to anti-BDCA-2 antibodies or antigen-binding fragments thereof. The present disclosure also relates to compositions comprising the anti-BDCA-2 antibody or antigen-binding fragment thereof, nucleic acids encoding the anti-BDCA-2 antibody or antigen-binding fragment thereof, vectors and host cells comprising the nucleic acids. The present disclosure further relates to therapeutic uses of the anti-BDCA-2 antibodies or antigen-binding fragments thereof and the pharmaceutical compositions comprising the same.

### BACKGROUND

Plasmacytoid dendritic cells (pDCs) are a unique subset of dendritic cells. After differentiation from the bone marrow, they enter the bloodstream, with some residing in the T zone and near the B zone of secondary lymphoid organs. Under conditions such as viral infection, pDCs massively flow into secondary lymphoid organs, residing in the T and B zones (PMID: 18787223, Blood. 2008 Dec 1;112(12):4598-608), and can rapidly secrete large amounts of type I IFN to combat viral infection. pDCs mainly rely on intracellular toll-like receptors (TLRs) 7, 8, and 9 to sense invading viruses and bacteria. Activation of these receptors can induce cytokine production. The body's nucleic acids and invading exogenous pathogens are structurally similar; TLRs activate pDCs after recognizing their own nucleic acid antigens. pDCs appear to be involved in the pathogenesis of autoimmune diseases and tumors. Studies have shown that invasive pDCs in breast and ovarian cancers are associated with poor clinical outcomes, indicating that pDCs are also involved in the progression of these cancers. pDCs are also directly involved in the progression of multiple myeloma (MM).

Type I interferon in the body is mainly induced by pDCs under external stimuli, such as viral infection, dsDNA, and ssRNA (PMID: 15987599, Cell Res. 2005 Jun;15 (6):407-22). pDCs and the interferon they produce are closely related to the pathogenesis of autoimmune diseases such as systemic lupus erythematosus (SLE), scleroderma, polymyositis, dermatomyositis, psoriasis, Sjogren's syndrome, rheumatoid arthritis, Graves' disease, and Hashimoto's disease (PMID: 32489664, Clin Transl Immunology. 2020 May 26;9 (5):e1139). In psoriasis and SLE patients, large amounts of pDC deposition are visible on the skin, leading to the activation of myxoviral resistance protein 1 (MxA) and other substances, which in turn cause symptoms (PMID: 26837058, Exp Dermatol. 2016 Jun; 25(6):415-21). In SLE patients, autoantigens can activate intracellular TLRs in pDC cells, leading to the production of type I interferon and other cytokines (PMID: 17979852, Immunol Rev. 2007 Dec; 220:251-69). The type I interferon receptor antagonist Saphnero^{®} (anifrolumab) has been approved by the FDA for the treatment of SLE (PMID: 32237942, Immunotherapy. 2020 Apr; 12(5):275-286).

Blood Dendritic Cell Antigen 2 (BDCA-2, also known as CD303 or CLEC4C) is a pattern recognition receptor (PRR) specifically expressed on the surface of plasma cell-like dendritic cells. It belongs to type 2 lectin receptors and can recognize certain sugar-containing or glycosylated proteins (PMID: 25995448, J Biol Chem. 2015 Jul 3; 290(27):16759-71). Known BDCA-2 specific antibodies, such as 24F4A (BIIB059), induce BDCA2 internalization upon binding to BDCA2, thereby inhibiting the production of type I interferon and co-stimulatory molecules. They can also utilize the Fc terminus of the antibody to exert ADCC and CDC effects to kill pDCs (PMID: 25762615, EMBO Mol Med. 2015 Apr; 7(4):464-76; PMID: 20673884, Cell Immunol. 2010; 265(1):15-22; US9902775B2). BDCA-2 specific antibodies can reduce pDC levels and inhibit downstream signaling of type I interferon in cynomolgus monkeys, and in early clinical studies, they have shown improvement in the clinical symptoms of SLE patients (PMID: 30645203, J Clin Invest. 2019 Mar 1;129(3):1359-1371).

However, there remains an urgent need in the field of SLE treatment for novel antibodies that specifically recognize BDCA-2 to optimize treatment for SLE patients, alleviate their symptoms, and improve the clinical efficacy of SLE. This application addresses this need.

### SUMMARY OF THE INVENTION

In a first aspect, the present disclosure provides an antibody or antigen-binding fragment thereof targeting BDCA-2, which has the following advantages:
(1) High affinity binding to human BDCA-2 and target cells expressing human BDCA-2;
(2) High affinity binding to cynomolgus monkey BDCA-2;
(3) Ability to induce internalization of BDCA2;
(4) Ability to inhibit CpG and immune complex-induced secretion of type I interferon by pDC cells;
(5) Ability to induce a strong ADCC effect;
(6) Durable inhibition of BDCA-2 expression in cynomolgus monkeys.

The present disclosure provides murine anti-BDCA-2 antibodies, chimeric antibodies, and humanized anti-BDCA-2 antibodies that specifically bind to BDCA-2. It is anticipated that the anti-BDCA-2 antibodies of the present disclosure have low immunogenicity in human subjects and well tolerated by human subjects.

In one embodiment, the present disclosure provides an anti-BDCA-2 antibody or antigen-binding fragment thereof that binds to BDCA-2, comprising:
1) the three heavy chain CDRs (HCDR1, HCDR2, HCDR3) comprised in the heavy chain variable region as set forth in SEQ ID NO: 7 and/or the three light chain CDRs (LCDR1, LCDR2, LCDR3) comprised in the light chain variable region as set forth in SEQ ID NO: 8;
2) the three heavy chain CDRs (HCDR1, HCDR2, HCDR3) comprised in the heavy chain variable region as set forth in SEQ ID NO: 17 and/or the three light chain CDRs (LCDR1, LCDR2, LCDR3) comprised in the light chain variable region as set forth in SEQ ID NO: 18.

In one embodiment, the present disclosure provides an anti-BDCA-2 antibody or antigen-binding fragment thereof that binds to BDCA-2, comprising:
1) heavy chain variable region CDRs (HCDR1, HCDR2, HCDR3) and/or light chain variable region CDRs (LCDR1, LCDR2, LCDR3), wherein HCDR1 comprises the sequence as set forth in SEQ ID NO: 1, or a sequence comprising one or more amino acid substitutions (*e.g*., conservative substitutions), deletions or insertions, or any combination thereof, relative to SEQ ID NO: 1, or consists of SEQ ID NO: 1; HCDR2 comprises the sequence as set forth in SEQ ID NO: 2, or a sequence comprising one or more amino acid substitutions (*e.g*., conservative substitutions), deletions or insertions, or any combination thereof, relative to SEQ ID NO: 2, or consists of SEQ ID NO: 2; HCDR3 comprises the sequence as set forth in SEQ ID NO: 3, or a sequence comprising one or more amino acid substitutions (*e.g*., conservative substitutions), deletions or insertions, or any combination thereof, relative to SEQ ID NO: 3, or consists of SEQ ID NO: 3; LCDR1 comprises the sequence as set forth in SEQ ID NO: 4, or a sequence comprising one or more amino acid substitutions (*e.g*., conservative substitutions), deletions or insertions, or any combination thereof, relative to SEQ ID NO: 4, or consists of SEQ ID NO: 4; LCDR2 comprises the sequence as set forth in SEQ ID NO: 5, or a sequence comprising one or more amino acid substitutions (*e.g*., conservative substitutions), deletions or insertions, or any combination thereof, relative to SEQ ID NO: 5, or consists of SEQ ID NO: 5; LCDR3 comprises the sequence as set forth in SEQ ID NO: 6, or a sequence comprising one or more amino acid substitutions (*e.g*., conservative substitutions), deletions or insertions, or any combination thereof, relative to SEQ ID NO: 6, or consists of SEQ ID NO: 6; and/or
2) heavy chain variable region CDRs (HCDR1, HCDR2, HCDR3) and/or light chain variable region CDRs (LCDR1, LCDR2, LCDR3), wherein HCDR1 comprises the sequence as set forth in SEQ ID NO: 11, or a sequence comprising one or more amino acid substitutions (*e.g.*, conservative substitutions), deletions or insertions, or any combination thereof, relative to SEQ ID NO: 11, or consists of SEQ ID NO: 11; HCDR2 comprises the sequence as set forth in SEQ ID NO: 12, or a sequence comprising one or more amino acid substitutions (*e.g*., conservative substitutions), deletions or insertions, or any combination thereof, relative to SEQ ID NO: 12, or consists of SEQ ID NO: 12; HCDR3 comprises the sequence as set forth in SEQ ID NO: 13, or a sequence comprising one or more amino acid substitutions (*e.g*., conservative substitutions), deletions or insertions, or any combination thereof, relative to SEQ ID NO: 13, or consists of SEQ ID NO: 13; LCDR1 comprises the sequence as set forth in SEQ ID NO: 14, or a sequence comprising one or more amino acid substitutions (*e.g*., conservative substitutions), deletions or insertions, or any combination thereof, relative to SEQ ID NO: 14, or consists of SEQ ID NO: 14; LCDR2 comprises the sequence as set forth in SEQ ID NO: 15, or a sequence comprising one or more amino acid substitutions (*e.g*., conservative substitutions), deletions or insertions, or any combination thereof, relative to SEQ ID NO: 15, or consists of SEQ ID NO: 15; LCDR3 comprises the sequence as set forth in SEQ ID NO: 16, or a sequence comprising one or more amino acid substitutions (*e.g*., conservative substitutions), deletions or insertions, or any combination thereof, relative to SEQ ID NO: 16, or consists of SEQ ID NO: 16.

In one embodiment, the present disclosure provides an anti-BDCA-2 antibody or antigen-binding fragment thereof that binds to BDCA-2, comprising a heavy chain variable region, wherein:
1) the heavy chain variable region comprises the amino acid sequence as set forth in SEQ ID NO: 7 or a sequence comprising one or more amino acid substitutions (*e.g*., conservative substitutions), deletions or insertions, or any combination thereof, relative to SEQ ID NO: 7, or an amino acid sequence having at least 90%, 91%, 92%, 93%, 94%, 95%, 96%, 97%, 98%, or 99% identity with the amino acid sequence of SEQ ID NO: 7, or consists of SEQ ID NO: 7;
2) the heavy chain variable region comprises the amino acid sequence as set forth in SEQ ID NO: 17 or a sequence comprising one or more amino acid substitutions (*e.g*., conservative substitutions), deletions or insertions, or any combination thereof, relative to SEQ ID NO: 17, or an amino acid sequence having at least 90%, 91%, 92%, 93%, 94%, 95%, 96%, 97%, 98%, or 99% identity with the amino acid sequence of SEQ ID NO: 17, or consists of SEQ ID NO: 17;
3) the heavy chain variable region comprises the amino acid sequence as set forth in SEQ ID NO: 19 or a sequence comprising one or more amino acid substitutions (*e.g*., conservative substitutions), deletions or insertions, or any combination thereof, relative to SEQ ID NO: 19, or an amino acid sequence having at least 90%, 91%, 92%, 93%, 94%, 95%, 96%, 97%, 98%, or 99% identity with the amino acid sequence of SEQ ID NO: 19, or consists of SEQ ID NO: 19;
4) the heavy chain variable region comprises the amino acid sequence as set forth in SEQ ID NO: 20 or a sequence comprising one or more amino acid substitutions (*e.g*., conservative substitutions), deletions or insertions, or any combination thereof, relative to SEQ ID NO: 20, or an amino acid sequence having at least 90%, 91%, 92%, 93%, 94%, 95%, 96%, 97%, 98%, or 99% identity with the amino acid sequence of SEQ ID NO: 20, or consists of SEQ ID NO: 20;
5) the heavy chain variable region comprises the amino acid sequence as set forth in SEQ ID NO: 21 or a sequence comprising one or more amino acid substitutions (*e.g*., conservative substitutions), deletions or insertions, or any combination thereof, relative to SEQ ID NO: 21, or an amino acid sequence having at least 90%, 91%, 92%, 93%, 94%, 95%, 96%, 97%, 98%, or 99% identity with the amino acid sequence of SEQ ID NO: 21, or consists of SEQ ID NO: 21;
6) the heavy chain variable region comprises the amino acid sequence as set forth in SEQ ID NO: 26 or a sequence comprising one or more amino acid substitutions (*e.g*., conservative substitutions), deletions or insertions, or any combination thereof, relative to SEQ ID NO: 26, or an amino acid sequence having at least 90%, 91%, 92%, 93%, 94%, 95%, 96%, 97%, 98%, or 99% identity with the amino acid sequence of SEQ ID NO: 26, or consists of SEQ ID NO: 26;
7) the heavy chain variable region comprises the amino acid sequence as set forth in SEQ ID NO: 27 or a sequence comprising one or more amino acid substitutions (*e.g*., conservative substitutions), deletions or insertions, or any combination thereof, relative to SEQ ID NO: 27, or an amino acid sequence having at least 90%, 91%, 92%, 93%, 94%, 95%, 96%, 97%, 98%, or 99% identity with the amino acid sequence of SEQ ID NO: 27, or consists of SEQ ID NO: 27;
8) the heavy chain variable region comprises the amino acid sequence as set forth in SEQ ID NO: 28 or a sequence comprising one or more amino acid substitutions (*e.g*., conservative substitutions), deletions or insertions, or any combination thereof, relative to SEQ ID NO: 28, or an amino acid sequence having at least 90%, 91%, 92%, 93%, 94%, 95%, 96%, 97%, 98%, or 99% identity with the amino acid sequence of SEQ ID NO: 28, or consists of SEQ ID NO: 28; or
9) the heavy chain variable region comprises the amino acid sequence as set forth in SEQ ID NO: 29 or a sequence comprising one or more amino acid substitutions (*e.g*., conservative substitutions), deletions or insertions, or any combination thereof, relative to SEQ ID NO: 29, or an amino acid sequence having at least 90%, 91%, 92%, 93%, 94%, 95%, 96%, 97%, 98%, or 99% identity with the amino acid sequence of SEQ ID NO: 29, or consists of SEQ ID NO: 29.

In one embodiment, the present disclosure provides an anti-BDCA-2 antibody or antigen-binding fragment thereof that binds to BDCA-2, comprising a light chain variable region, wherein:
1) the light chain variable region comprises the amino acid sequence as set forth in SEQ ID NO: 8 or a sequence comprising one or more amino acid substitutions (*e.g*., conservative substitutions), deletions or insertions, or any combination thereof, relative to SEQ ID NO: 8, or an amino acid sequence having at least 90%, 91%, 92%, 93%, 94%, 95%, 96%, 97%, 98%, or 99% identity with the amino acid sequence of SEQ ID NO: 8, or consists of SEQ ID NO: 8;
2) the light chain variable region comprises the amino acid sequence as set forth in SEQ ID NO: 18 or a sequence comprising one or more amino acid substitutions (*e.g*., conservative substitutions), deletions or insertions, or any combination thereof, relative to SEQ ID NO: 18, or an amino acid sequence having at least 90%, 91%, 92%, 93%, 94%, 95%, 96%, 97%, 98%, or 99% identity with the amino acid sequence of SEQ ID NO: 18, or consists of SEQ ID NO: 18;
3) the light chain variable region comprises the amino acid sequence as set forth in SEQ ID NO: 22 or a sequence comprising one or more amino acid substitutions (*e.g*., conservative substitutions), deletions or insertions, or any combination thereof, relative to SEQ ID NO: 22, or an amino acid sequence having at least 90%, 91%, 92%, 93%, 94%, 95%, 96%, 97%, 98%, or 99% identity with the amino acid sequence of SEQ ID NO: 22, or consists of SEQ ID NO: 22;
4) the light chain variable region comprises the amino acid sequence as set forth in SEQ ID NO: 23 or a sequence comprising one or more amino acid substitutions (*e.g*., conservative substitutions), deletions or insertions, or any combination thereof, relative to SEQ ID NO: 23, or an amino acid sequence having at least 90%, 91%, 92%, 93%, 94%, 95%, 96%, 97%, 98%, or 99% identity with the amino acid sequence of SEQ ID NO: 23, or consists of SEQ ID NO: 23;
5) the light chain variable region comprises the amino acid sequence as set forth in SEQ ID NO: 24 or a sequence comprising one or more amino acid substitutions (*e.g*., conservative substitutions), deletions or insertions, or any combination thereof, relative to SEQ ID NO: 24, or an amino acid sequence having at least 90%, 91%, 92%, 93%, 94%, 95%, 96%, 97%, 98%, or 99% identity with the amino acid sequence of SEQ ID NO: 24, or consists of SEQ ID NO: 24;
6) the light chain variable region comprises the amino acid sequence as set forth in SEQ ID NO: 25 or a sequence comprising one or more amino acid substitutions (*e.g*., conservative substitutions), deletions or insertions, or any combination thereof, relative to SEQ ID NO: 25, or an amino acid sequence having at least 90%, 91%, 92%, 93%, 94%, 95%, 96%, 97%, 98%, or 99% identity with the amino acid sequence of SEQ ID NO: 25, or consists of SEQ ID NO: 25;
7) the light chain variable region comprises the amino acid sequence as set forth in SEQ ID NO: 30 or a sequence comprising one or more amino acid substitutions (*e.g.*, conservative substitutions), deletions or insertions, or any combination thereof, relative to SEQ ID NO: 30, or an amino acid sequence having at least 90%, 91%, 92%, 93%, 94%, 95%, 96%, 97%, 98%, or 99% identity with the amino acid sequence of SEQ ID NO: 30, or consists of SEQ ID NO: 30;
8) the light chain variable region comprises the amino acid sequence as set forth in SEQ ID NO: 31 or a sequence comprising one or more amino acid substitutions (*e.g*., conservative substitutions), deletions or insertions, or any combination thereof, relative to SEQ ID NO: 31, or an amino acid sequence having at least 90%, 91%, 92%, 93%, 94%, 95%, 96%, 97%, 98%, or 99% identity with the amino acid sequence of SEQ ID NO: 31, or consists of SEQ ID NO: 31; or
9) the light chain variable region comprises the amino acid sequence as set forth in SEQ ID NO: 32 or a sequence comprising one or more amino acid substitutions (*e.g*., conservative substitutions), deletions or insertions, or any combination thereof, relative to SEQ ID NO: 32, or an amino acid sequence having at least 90%, 91%, 92%, 93%, 94%, 95%, 96%, 97%, 98%, or 99% identity with the amino acid sequence of SEQ ID NO: 32, or consists of SEQ ID NO: 32.

In another embodiment, the present disclosure provides an anti-BDCA-2 antibody or antigen-binding fragment thereof that binds to BDCA-2, comprising a heavy chain variable region and/or a light chain variable region, wherein:
1) the heavy chain variable region comprises the amino acid sequence as set forth in SEQ ID NO: 7 or a sequence comprising one or more amino acid substitutions (*e.g*., conservative substitutions), deletions or insertions, or any combination thereof, relative to SEQ ID NO: 7, or an amino acid sequence having at least 90%, 91%, 92%, 93%, 94%, 95%, 96%, 97%, 98%, or 99% identity with the amino acid sequence of SEQ ID NO: 7, or consists of SEQ ID NO: 7; and the light chain variable region comprises the amino acid sequence as set forth in SEQ ID NO: 8 or a sequence comprising one or more amino acid substitutions (*e.g*., conservative substitutions), deletions or insertions, or any combination thereof, relative to SEQ ID NO: 8, or an amino acid sequence having at least 90%, 91%, 92%, 93%, 94%, 95%, 96%, 97%, 98%, or 99% identity with the amino acid sequence of SEQ ID NO: 8, or consists of SEQ ID NO: 8;
2) the heavy chain variable region comprises the amino acid sequence as set forth in SEQ ID NO: 17 or a sequence comprising one or more amino acid substitutions (*e.g*., conservative substitutions), deletions or insertions, or any combination thereof, relative to SEQ ID NO: 17, or an amino acid sequence having at least 90%, 91%, 92%, 93%, 94%, 95%, 96%, 97%, 98%, or 99% identity with the amino acid sequence of SEQ ID NO: 17, or consists of SEQ ID NO: 17; and the light chain variable region comprises the amino acid sequence as set forth in SEQ ID NO: 18 or a sequence comprising one or more amino acid substitutions (*e.g*., conservative substitutions), deletions or insertions, or any combination thereof, relative to SEQ ID NO: 18, or an amino acid sequence having at least 90%, 91%, 92%, 93%, 94%, 95%, 96%, 97%, 98%, or 99% identity with the amino acid sequence of SEQ ID NO: 18, or consists of SEQ ID NO: 18;
3) the heavy chain variable region comprises the amino acid sequence as set forth in SEQ ID NO: 19 or a sequence comprising one or more amino acid substitutions (*e.g*., conservative substitutions), deletions or insertions, or any combination thereof, relative to SEQ ID NO: 19, or an amino acid sequence having at least 90%, 91%, 92%, 93%, 94%, 95%, 96%, 97%, 98%, or 99% identity with the amino acid sequence of SEQ ID NO: 19, or consists of SEQ ID NO: 19; and the light chain variable region comprises the amino acid sequence as set forth in SEQ ID NO: 22 or a sequence comprising one or more amino acid substitutions (*e.g*., conservative substitutions), deletions or insertions, or any combination thereof, relative to SEQ ID NO: 22, or an amino acid sequence having at least 90%, 91%, 92%, 93%, 94%, 95%, 96%, 97%, 98%, or 99% identity with the amino acid sequence of SEQ ID NO: 22, or consists of SEQ ID NO: 22;
4) the heavy chain variable region comprises the amino acid sequence as set forth in SEQ ID NO: 19 or a sequence comprising one or more amino acid substitutions (*e.g*., conservative substitutions), deletions or insertions, or any combination thereof, relative to SEQ ID NO: 19, or an amino acid sequence having at least 90%, 91%, 92%, 93%, 94%, 95%, 96%, 97%, 98%, or 99% identity with the amino acid sequence of SEQ ID NO: 19, or consists of SEQ ID NO: 19; and the light chain variable region comprises the amino acid sequence as set forth in SEQ ID NO: 23 or a sequence comprising one or more amino acid substitutions (*e.g*., conservative substitutions), deletions or insertions, or any combination thereof, relative to SEQ ID NO: 23, or an amino acid sequence having at least 90%, 91%, 92%, 93%, 94%, 95%, 96%, 97%, 98%, or 99% identity with the amino acid sequence of SEQ ID NO: 23, or consists of SEQ ID NO: 23;
5) the heavy chain variable region comprises the amino acid sequence as set forth in SEQ ID NO: 19 or a sequence comprising one or more amino acid substitutions (*e.g*., conservative substitutions), deletions or insertions, or any combination thereof, relative to SEQ ID NO: 19, or an amino acid sequence having at least 90%, 91%, 92%, 93%, 94%, 95%, 96%, 97%, 98%, or 99% identity with the amino acid sequence of SEQ ID NO: 19, or consists of SEQ ID NO: 19; and the light chain variable region comprises the amino acid sequence as set forth in SEQ ID NO: 24 or a sequence comprising one or more amino acid substitutions (*e.g*., conservative substitutions), deletions or insertions, or any combination thereof, relative to SEQ ID NO: 24, or an amino acid sequence having at least 90%, 91%, 92%, 93%, 94%, 95%, 96%, 97%, 98%, or 99% identity with the amino acid sequence of SEQ ID NO: 24, or consists of SEQ ID NO: 24;
6) the heavy chain variable region comprises the amino acid sequence as set forth in SEQ ID NO: 19 or a sequence comprising one or more amino acid substitutions (*e.g*., conservative substitutions), deletions or insertions, or any combination thereof, relative to SEQ ID NO: 19, or an amino acid sequence having at least 90%, 91%, 92%, 93%, 94%, 95%, 96%, 97%, 98%, or 99% identity with the amino acid sequence of SEQ ID NO: 19, or consists of SEQ ID NO: 19; and the light chain variable region comprises the amino acid sequence as set forth in SEQ ID NO: 25 or a sequence comprising one or more amino acid substitutions (*e.g*., conservative substitutions), deletions or insertions, or any combination thereof, relative to SEQ ID NO: 25, or an amino acid sequence having at least 90%, 91%, 92%, 93%, 94%, 95%, 96%, 97%, 98%, or 99% identity with the amino acid sequence of SEQ ID NO: 25, or consists of SEQ ID NO: 25;
7) the heavy chain variable region comprises the amino acid sequence as set forth in SEQ ID NO: 20 or a sequence comprising one or more amino acid substitutions (*e.g.*, conservative substitutions), deletions or insertions, or any combination thereof, relative to SEQ ID NO: 20, or an amino acid sequence having at least 90%, 91%, 92%, 93%, 94%, 95%, 96%, 97%, 98%, or 99% identity with the amino acid sequence of SEQ ID NO: 20, or consists of SEQ ID NO: 20; and the light chain variable region comprises the amino acid sequence as set forth in SEQ ID NO: 22 or a sequence comprising one or more amino acid substitutions (*e.g*., conservative substitutions), deletions or insertions, or any combination thereof, relative to SEQ ID NO: 22, or an amino acid sequence having at least 90%, 91%, 92%, 93%, 94%, 95%, 96%, 97%, 98%, or 99% identity with the amino acid sequence of SEQ ID NO: 22, or consists of SEQ ID NO: 22;
8) the heavy chain variable region comprises the amino acid sequence as set forth in SEQ ID NO: 20 or a sequence comprising one or more amino acid substitutions (*e.g*., conservative substitutions), deletions or insertions, or any combination thereof, relative to SEQ ID NO: 20, or an amino acid sequence having at least 90%, 91%, 92%, 93%, 94%, 95%, 96%, 97%, 98%, or 99% identity with the amino acid sequence of SEQ ID NO: 20, or consists of SEQ ID NO: 20; and the light chain variable region comprises the amino acid sequence as set forth in SEQ ID NO: 23 or a sequence comprising one or more amino acid substitutions (*e.g*., conservative substitutions), deletions or insertions, or any combination thereof, relative to SEQ ID NO: 23, or an amino acid sequence having at least 90%, 91%, 92%, 93%, 94%, 95%, 96%, 97%, 98%, or 99% identity with the amino acid sequence of SEQ ID NO: 23, or consists of SEQ ID NO: 23;
9) the heavy chain variable region comprises the amino acid sequence as set forth in SEQ ID NO: 20 or a sequence comprising one or more amino acid substitutions (*e.g*., conservative substitutions), deletions or insertions, or any combination thereof, relative to SEQ ID NO: 20, or an amino acid sequence having at least 90%, 91%, 92%, 93%, 94%, 95%, 96%, 97%, 98%, or 99% identity with the amino acid sequence of SEQ ID NO: 20, or consists of SEQ ID NO: 20; and the light chain variable region comprises the amino acid sequence as set forth in SEQ ID NO: 24 or a sequence comprising one or more amino acid substitutions (*e.g*., conservative substitutions), deletions or insertions, or any combination thereof, relative to SEQ ID NO: 24, or an amino acid sequence having at least 90%, 91%, 92%, 93%, 94%, 95%, 96%, 97%, 98%, or 99% identity with the amino acid sequence of SEQ ID NO: 24, or consists of SEQ ID NO: 24;
10) the heavy chain variable region comprises the amino acid sequence as set forth in SEQ ID NO: 20 or a sequence comprising one or more amino acid substitutions (*e.g*., conservative substitutions), deletions or insertions, or any combination thereof, relative to SEQ ID NO: 20, or an amino acid sequence having at least 90%, 91%, 92%, 93%, 94%, 95%, 96%, 97%, 98%, or 99% identity with the amino acid sequence of SEQ ID NO: 20, or consists of SEQ ID NO: 20; and the light chain variable region comprises the amino acid sequence as set forth in SEQ ID NO: 25 or a sequence comprising one or more amino acid substitutions (*e.g*., conservative substitutions), deletions or insertions, or any combination thereof, relative to SEQ ID NO: 25, or an amino acid sequence having at least 90%, 91%, 92%, 93%, 94%, 95%, 96%, 97%, 98%, or 99% identity with the amino acid sequence of SEQ ID NO: 25, or consists of SEQ ID NO: 25;
11) the heavy chain variable region comprises the amino acid sequence as set forth in SEQ ID NO: 21 or a sequence comprising one or more amino acid substitutions (*e.g*., conservative substitutions), deletions or insertions, or any combination thereof, relative to SEQ ID NO: 21, or an amino acid sequence having at least 90%, 91%, 92%, 93%, 94%, 95%, 96%, 97%, 98%, or 99% identity with the amino acid sequence of SEQ ID NO: 21, or consists of SEQ ID NO: 21; and the light chain variable region comprises the amino acid sequence as set forth in SEQ ID NO: 22 or a sequence comprising one or more amino acid substitutions (*e.g*., conservative substitutions), deletions or insertions, or any combination thereof, relative to SEQ ID NO: 22, or an amino acid sequence having at least 90%, 91%, 92%, 93%, 94%, 95%, 96%, 97%, 98%, or 99% identity with the amino acid sequence of SEQ ID NO: 22, or consists of SEQ ID NO: 22;
12) the heavy chain variable region comprises the amino acid sequence as set forth in SEQ ID NO: 21 or a sequence comprising one or more amino acid substitutions (*e.g*., conservative substitutions), deletions or insertions, or any combination thereof, relative to SEQ ID NO: 21, or an amino acid sequence having at least 90%, 91%, 92%, 93%, 94%, 95%, 96%, 97%, 98%, or 99% identity with the amino acid sequence of SEQ ID NO: 21, or consists of SEQ ID NO: 21; and the light chain variable region comprises the amino acid sequence as set forth in SEQ ID NO: 23 or a sequence comprising one or more amino acid substitutions (*e.g*., conservative substitutions), deletions or insertions, or any combination thereof, relative to SEQ ID NO: 23, or an amino acid sequence having at least 90%, 91%, 92%, 93%, 94%, 95%, 96%, 97%, 98%, or 99% identity with the amino acid sequence of SEQ ID NO: 23, or consists of SEQ ID NO: 23;
13) the heavy chain variable region comprises the amino acid sequence as set forth in SEQ ID NO: 21 or a sequence comprising one or more amino acid substitutions (*e.g*., conservative substitutions), deletions or insertions, or any combination thereof, relative to SEQ ID NO: 21, or an amino acid sequence having at least 90%, 91%, 92%, 93%, 94%, 95%, 96%, 97%, 98%, or 99% identity with the amino acid sequence of SEQ ID NO: 21, or consists of SEQ ID NO: 21; and the light chain variable region comprises the amino acid sequence as set forth in SEQ ID NO: 24 or a sequence comprising one or more amino acid substitutions (*e.g*., conservative substitutions), deletions or insertions, or any combination thereof, relative to SEQ ID NO: 24, or an amino acid sequence having at least 90%, 91%, 92%, 93%, 94%, 95%, 96%, 97%, 98%, or 99% identity with the amino acid sequence of SEQ ID NO: 24, or consists of SEQ ID NO: 24;
14) the heavy chain variable region comprises the amino acid sequence as set forth in SEQ ID NO: 21 or a sequence comprising one or more amino acid substitutions (*e.g*., conservative substitutions), deletions or insertions, or any combination thereof, relative to SEQ ID NO: 21, or an amino acid sequence having at least 90%, 91%, 92%, 93%, 94%, 95%, 96%, 97%, 98%, or 99% identity with the amino acid sequence of SEQ ID NO: 21, or consists of SEQ ID NO: 21; and the light chain variable region comprises the amino acid sequence as set forth in SEQ ID NO: 25 or a sequence comprising one or more amino acid substitutions (*e.g*., conservative substitutions), deletions or insertions, or any combination thereof, relative to SEQ ID NO: 25, or an amino acid sequence having at least 90%, 91%, 92%, 93%, 94%, 95%, 96%, 97%, 98%, or 99% identity with the amino acid sequence of SEQ ID NO: 25, or consists of SEQ ID NO: 25;
15) the heavy chain variable region comprises the amino acid sequence as set forth in SEQ ID NO: 26 or a sequence comprising one or more amino acid substitutions (*e.g*., conservative substitutions), deletions or insertions, or any combination thereof, relative to SEQ ID NO: 26, or an amino acid sequence having at least 90%, 91%, 92%, 93%, 94%, 95%, 96%, 97%, 98%, or 99% identity with the amino acid sequence of SEQ ID NO: 26, or consists of SEQ ID NO: 26; and the light chain variable region comprises the amino acid sequence as set forth in SEQ ID NO: 30 or a sequence comprising one or more amino acid substitutions (*e.g*., conservative substitutions), deletions or insertions, or any combination thereof, relative to SEQ ID NO: 30, or an amino acid sequence having at least 90%, 91%, 92%, 93%, 94%, 95%, 96%, 97%, 98%, or 99% identity with the amino acid sequence of SEQ ID NO: 30, or consists of SEQ ID NO: 30;
16) the heavy chain variable region comprises the amino acid sequence as set forth in SEQ ID NO: 26 or a sequence comprising one or more amino acid substitutions (*e.g*., conservative substitutions), deletions or insertions, or any combination thereof, relative to SEQ ID NO: 26, or an amino acid sequence having at least 90%, 91%, 92%, 93%, 94%, 95%, 96%, 97%, 98%, or 99% identity with the amino acid sequence of SEQ ID NO: 26, or consists of SEQ ID NO: 26; and the light chain variable region comprises the amino acid sequence as set forth in SEQ ID NO: 31 or a sequence comprising one or more amino acid substitutions (*e.g*., conservative substitutions), deletions or insertions, or any combination thereof, relative to SEQ ID NO: 31, or an amino acid sequence having at least 90%, 91%, 92%, 93%, 94%, 95%, 96%, 97%, 98%, or 99% identity with the amino acid sequence of SEQ ID NO: 31, or consists of SEQ ID NO: 31;
17) the heavy chain variable region comprises the amino acid sequence as set forth in SEQ ID NO: 26 or a sequence comprising one or more amino acid substitutions (*e.g*., conservative substitutions), deletions or insertions, or any combination thereof, relative to SEQ ID NO: 26, or an amino acid sequence having at least 90%, 91%, 92%, 93%, 94%, 95%, 96%, 97%, 98%, or 99% identity with the amino acid sequence of SEQ ID NO: 26, or consists of SEQ ID NO: 26; and the light chain variable region comprises the amino acid sequence as set forth in SEQ ID NO: 32 or a sequence comprising one or more amino acid substitutions (*e.g*., conservative substitutions), deletions or insertions, or any combination thereof, relative to SEQ ID NO: 32, or an amino acid sequence having at least 90%, 91%, 92%, 93%, 94%, 95%, 96%, 97%, 98%, or 99% identity with the amino acid sequence of SEQ ID NO: 32, or consists of SEQ ID NO: 32;
18) the heavy chain variable region comprises the amino acid sequence as set forth in SEQ ID NO: 27 or a sequence comprising one or more amino acid substitutions (*e.g*., conservative substitutions), deletions or insertions, or any combination thereof, relative to SEQ ID NO: 27, or an amino acid sequence having at least 90%, 91%, 92%, 93%, 94%, 95%, 96%, 97%, 98%, or 99% identity with the amino acid sequence of SEQ ID NO: 27, or consists of SEQ ID NO: 27; and the light chain variable region comprises the amino acid sequence as set forth in SEQ ID NO: 30 or a sequence comprising one or more amino acid substitutions (*e.g*., conservative substitutions), deletions or insertions, or any combination thereof, relative to SEQ ID NO: 30, or an amino acid sequence having at least 90%, 91%, 92%, 93%, 94%, 95%, 96%, 97%, 98%, or 99% identity with the amino acid sequence of SEQ ID NO: 30, or consists of SEQ ID NO: 30;
19) the heavy chain variable region comprises the amino acid sequence as set forth in SEQ ID NO: 27 or a sequence comprising one or more amino acid substitutions (*e.g*., conservative substitutions), deletions or insertions, or any combination thereof, relative to SEQ ID NO: 27, or an amino acid sequence having at least 90%, 91%, 92%, 93%, 94%, 95%, 96%, 97%, 98%, or 99% identity with the amino acid sequence of SEQ ID NO: 27, or consists of SEQ ID NO: 27; and the light chain variable region comprises the amino acid sequence as set forth in SEQ ID NO: 31 or a sequence comprising one or more amino acid substitutions (*e.g*., conservative substitutions), deletions or insertions, or any combination thereof, relative to SEQ ID NO: 31, or an amino acid sequence having at least 90%, 91%, 92%, 93%, 94%, 95%, 96%, 97%, 98%, or 99% identity with the amino acid sequence of SEQ ID NO: 31, or consists of SEQ ID NO: 31;
20) the heavy chain variable region comprises the amino acid sequence as set forth in SEQ ID NO: 27 or a sequence comprising one or more amino acid substitutions (*e.g*., conservative substitutions), deletions or insertions, or any combination thereof, relative to SEQ ID NO: 27, or an amino acid sequence having at least 90%, 91%, 92%, 93%, 94%, 95%, 96%, 97%, 98%, or 99% identity with the amino acid sequence of SEQ ID NO: 27, or consists of SEQ ID NO: 27; and the light chain variable region comprises the amino acid sequence as set forth in SEQ ID NO: 32 or a sequence comprising one or more amino acid substitutions (*e.g*., conservative substitutions), deletions or insertions, or any combination thereof, relative to SEQ ID NO: 32, or an amino acid sequence having at least 90%, 91%, 92%, 93%, 94%, 95%, 96%, 97%, 98%, or 99% identity with the amino acid sequence of SEQ ID NO: 32, or consists of SEQ ID NO: 32;
21) the heavy chain variable region comprises the amino acid sequence as set forth in SEQ ID NO: 28 or a sequence comprising one or more amino acid substitutions (*e.g*., conservative substitutions), deletions or insertions, or any combination thereof, relative to SEQ ID NO: 28, or an amino acid sequence having at least 90%, 91%, 92%, 93%, 94%, 95%, 96%, 97%, 98%, or 99% identity with the amino acid sequence of SEQ ID NO: 28, or consists of SEQ ID NO: 28; and the light chain variable region comprises the amino acid sequence as set forth in SEQ ID NO: 30 or a sequence comprising one or more amino acid substitutions (*e.g*., conservative substitutions), deletions or insertions, or any combination thereof, relative to SEQ ID NO: 30, or an amino acid sequence having at least 90%, 91%, 92%, 93%, 94%, 95%, 96%, 97%, 98%, or 99% identity with the amino acid sequence of SEQ ID NO: 30, or consists of SEQ ID NO: 30;
22) the heavy chain variable region comprises the amino acid sequence as set forth in SEQ ID NO: 28 or a sequence comprising one or more amino acid substitutions (*e.g*., conservative substitutions), deletions or insertions, or any combination thereof, relative to SEQ ID NO: 28, or an amino acid sequence having at least 90%, 91%, 92%, 93%, 94%, 95%, 96%, 97%, 98%, or 99% identity with the amino acid sequence of SEQ ID NO: 28, or consists of SEQ ID NO: 28; and the light chain variable region comprises the amino acid sequence as set forth in SEQ ID NO: 31 or a sequence comprising one or more amino acid substitutions (*e.g*., conservative substitutions), deletions or insertions, or any combination thereof, relative to SEQ ID NO: 31, or an amino acid sequence having at least 90%, 91%, 92%, 93%, 94%, 95%, 96%, 97%, 98%, or 99% identity with the amino acid sequence of SEQ ID NO: 31, or consists of SEQ ID NO: 31;
23) the heavy chain variable region comprises the amino acid sequence as set forth in SEQ ID NO: 28 or a sequence comprising one or more amino acid substitutions (*e.g*., conservative substitutions), deletions or insertions, or any combination thereof, relative to SEQ ID NO: 28, or an amino acid sequence having at least 90%, 91%, 92%, 93%, 94%, 95%, 96%, 97%, 98%, or 99% identity with the amino acid sequence of SEQ ID NO: 28, or consists of SEQ ID NO: 28; and the light chain variable region comprises the amino acid sequence as set forth in SEQ ID NO: 32 or a sequence comprising one or more amino acid substitutions (*e.g*., conservative substitutions), deletions or insertions, or any combination thereof, relative to SEQ ID NO: 32, or an amino acid sequence having at least 90%, 91%, 92%, 93%, 94%, 95%, 96%, 97%, 98%, or 99% identity with the amino acid sequence of SEQ ID NO: 32, or consists of SEQ ID NO: 32;
24) the heavy chain variable region comprises the amino acid sequence as set forth in SEQ ID NO: 29 or a sequence comprising one or more amino acid substitutions (*e.g*., conservative substitutions), deletions or insertions, or any combination thereof, relative to SEQ ID NO: 29, or an amino acid sequence having at least 90%, 91%, 92%, 93%, 94%, 95%, 96%, 97%, 98%, or 99% identity with the amino acid sequence of SEQ ID NO: 29, or consists of SEQ ID NO: 29; and the light chain variable region comprises the amino acid sequence as set forth in SEQ ID NO: 30 or a sequence comprising one or more amino acid substitutions (*e.g*., conservative substitutions), deletions or insertions, or any combination thereof, relative to SEQ ID NO: 30, or an amino acid sequence having at least 90%, 91%, 92%, 93%, 94%, 95%, 96%, 97%, 98%, or 99% identity with the amino acid sequence of SEQ ID NO: 30, or consists of SEQ ID NO: 30;
25) the heavy chain variable region comprises the amino acid sequence as set forth in SEQ ID NO: 29 or a sequence comprising one or more amino acid substitutions (*e.g*., conservative substitutions), deletions or insertions, or any combination thereof, relative to SEQ ID NO: 29, or an amino acid sequence having at least 90%, 91%, 92%, 93%, 94%, 95%, 96%, 97%, 98%, or 99% identity with the amino acid sequence of SEQ ID NO: 29, or consists of SEQ ID NO: 29; and the light chain variable region comprises the amino acid sequence as set forth in SEQ ID NO: 31 or a sequence comprising one or more amino acid substitutions (*e.g*., conservative substitutions), deletions or insertions, or any combination thereof, relative to SEQ ID NO: 31, or an amino acid sequence having at least 90%, 91%, 92%, 93%, 94%, 95%, 96%, 97%, 98%, or 99% identity with the amino acid sequence of SEQ ID NO: 31, or consists of SEQ ID NO: 31; or
26) the heavy chain variable region comprises the amino acid sequence as set forth in SEQ ID NO: 29 or a sequence comprising one or more amino acid substitutions (*e.g*., conservative substitutions), deletions or insertions, or any combination thereof, relative to SEQ ID NO: 29, or an amino acid sequence having at least 90%, 91%, 92%, 93%, 94%, 95%, 96%, 97%, 98%, or 99% identity with the amino acid sequence of SEQ ID NO: 29, or consists of SEQ ID NO: 29; and the light chain variable region comprises the amino acid sequence as set forth in SEQ ID NO: 32 or a sequence comprising one or more amino acid substitutions (*e.g*., conservative substitutions), deletions or insertions, or any combination thereof, relative to SEQ ID NO: 32, or an amino acid sequence having at least 90%, 91%, 92%, 93%, 94%, 95%, 96%, 97%, 98%, or 99% identity with the amino acid sequence of SEQ ID NO: 32, or consists of SEQ ID NO: 32.

In a specific embodiment, the present disclosure provides an anti-BDCA-2 antibody or antigen-binding fragment thereof that binds to BDCA-2, comprising a heavy chain variable region and/or a light chain variable region, wherein:
1) the heavy chain variable region comprises or consists of the amino acid sequence as set forth in SEQ ID NO: 7 and the light chain variable region comprises or consists of the amino acid sequence as set forth in SEQ ID NO: 8;
2) the heavy chain variable region comprises or consists of the amino acid sequence as set forth in SEQ ID NO: 17 and the light chain variable region comprises or consists of the amino acid sequence as set forth in SEQ ID NO: 18;
3) the heavy chain variable region comprises or consists of the amino acid sequence as set forth in SEQ ID NO: 19 and the light chain variable region comprises or consists of the amino acid sequence as set forth in SEQ ID NO: 22;
4) the heavy chain variable region comprises or consists of the amino acid sequence as set forth in SEQ ID NO: 19 and the light chain variable region comprises or consists of the amino acid sequence as set forth in SEQ ID NO: 23;
5) the heavy chain variable region comprises or consists of the amino acid sequence as set forth in SEQ ID NO: 19 and the light chain variable region comprises or consists of the amino acid sequence as set forth in SEQ ID NO: 24;
6) the heavy chain variable region comprises or consists of the amino acid sequence as set forth in SEQ ID NO: 19 and the light chain variable region comprises or consists of the amino acid sequence as set forth in SEQ ID NO: 25;
7) the heavy chain variable region comprises or consists of the amino acid sequence as set forth in SEQ ID NO: 20 and the light chain variable region comprises or consists of the amino acid sequence as set forth in SEQ ID NO: 22;
8) the heavy chain variable region comprises or consists of the amino acid sequence as set forth in SEQ ID NO: 20 and the light chain variable region comprises or consists of the amino acid sequence as set forth in SEQ ID NO: 23;
9) the heavy chain variable region comprises or consists of the amino acid sequence as set forth in SEQ ID NO: 20 and the light chain variable region comprises or consists of the amino acid sequence as set forth in SEQ ID NO: 24;
10) the heavy chain variable region comprises or consists of the amino acid sequence as set forth in SEQ ID NO: 20 and the light chain variable region comprises or consists of the amino acid sequence as set forth in SEQ ID NO: 25;
11) the heavy chain variable region comprises or consists of the amino acid sequence as set forth in SEQ ID NO: 21 and the light chain variable region comprises or consists of the amino acid sequence as set forth in SEQ ID NO: 22;
12) the heavy chain variable region comprises or consists of the amino acid sequence as set forth in SEQ ID NO: 21 and the light chain variable region comprises or consists of the amino acid sequence as set forth in SEQ ID NO: 23;
13) the heavy chain variable region comprises or consists of the amino acid sequence as set forth in SEQ ID NO: 21 and the light chain variable region comprises or consists of the amino acid sequence as set forth in SEQ ID NO: 24;
14) the heavy chain variable region comprises or consists of the amino acid sequence as set forth in SEQ ID NO: 21 and the light chain variable region comprises or consists of the amino acid sequence as set forth in SEQ ID NO: 25;
15) the heavy chain variable region comprises or consists of the amino acid sequence as set forth in SEQ ID NO: 26 and the light chain variable region comprises or consists of the amino acid sequence as set forth in SEQ ID NO: 30;
16) the heavy chain variable region comprises or consists of the amino acid sequence as set forth in SEQ ID NO: 26 and the light chain variable region comprises or consists of the amino acid sequence as set forth in SEQ ID NO: 31;
17) the heavy chain variable region comprises or consists of the amino acid sequence as set forth in SEQ ID NO: 26 and the light chain variable region comprises or consists of the amino acid sequence as set forth in SEQ ID NO: 32;
18) the heavy chain variable region comprises or consists of the amino acid sequence as set forth in SEQ ID NO: 27 and the light chain variable region comprises or consists of the amino acid sequence as set forth in SEQ ID NO: 30;
19) the heavy chain variable region comprises or consists of the amino acid sequence as set forth in SEQ ID NO: 27 and the light chain variable region comprises or consists of the amino acid sequence as set forth in SEQ ID NO: 31;
20) the heavy chain variable region comprises or consists of the amino acid sequence as set forth in SEQ ID NO: 27 and the light chain variable region comprises or consists of the amino acid sequence as set forth in SEQ ID NO: 32;
21) the heavy chain variable region comprises or consists of the amino acid sequence as set forth in SEQ ID NO: 28 and the light chain variable region comprises or consists of the amino acid sequence as set forth in SEQ ID NO: 30;
22) the heavy chain variable region comprises or consists of the amino acid sequence as set forth in SEQ ID NO: 28 and the light chain variable region comprises or consists of the amino acid sequence as set forth in SEQ ID NO: 31;
23) the heavy chain variable region comprises or consists of the amino acid sequence as set forth in SEQ ID NO: 28 and the light chain variable region comprises or consists of the amino acid sequence as set forth in SEQ ID NO: 32;
24) the heavy chain variable region comprises or consists of the amino acid sequence as set forth in SEQ ID NO: 29 and the light chain variable region comprises or consists of the amino acid sequence as set forth in SEQ ID NO: 30;
25) the heavy chain variable region comprises or consists of the amino acid sequence as set forth in SEQ ID NO: 29 and the light chain variable region comprises or consists of the amino acid sequence as set forth in SEQ ID NO: 31; or
26) the heavy chain variable region comprises or consists of the amino acid sequence as set forth in SEQ ID NO: 29 and the light chain variable region comprises or consists of the amino acid sequence as set forth in SEQ ID NO: 32.

In some embodiments, the antibody or antigen-binding fragment thereof further comprises heavy chain and/or light chain constant region sequences derived from a human antibody germline consensus sequence. In some embodiments, the heavy chain constant region is preferably derived from the constant region sequence of human IgG1, IgG2, IgG3, IgG 4. In another embodiment, the heavy chain constant region comprises or consists of the sequence of SEQ ID NO: 9. In one embodiment, the light chain constant region comprises or consists of SEQ ID NO: 10.

It should be understood that sequence variants of these constant region domains, for example comprising one or more amino acid modifications, wherein the amino acid position is identified by the EU indexing system of Kabat *et al.* (1991), may also be used.

In a specific embodiment, the present disclosure provides an anti-BDCA-2 antibody or antigen-binding fragment thereof that binds to BDCA-2, comprising a heavy chain and/or a light chain, wherein:
1) the heavy chain comprises the amino acid sequence as set forth in SEQ ID NO: 33 or a sequence comprising one or more amino acid substitutions (*e.g*., conservative substitutions), deletions or insertions, or any combination thereof, relative to SEQ ID NO: 33, or an amino acid sequence having at least 90%, 91%, 92%, 93%, 94%, 95%, 96%, 97%, 98%, or 99% identity with the amino acid sequence of SEQ ID NO: 33, or consists of SEQ ID NO: 33; and the light chain comprises the amino acid sequence as set forth in SEQ ID NO: 34 or a sequence comprising one or more amino acid substitutions (*e.g*., conservative substitutions), deletions or insertions, or any combination thereof, relative to SEQ ID NO: 34, or an amino acid sequence having at least 90%, 91%, 92%, 93%, 94%, 95%, 96%, 97%, 98%, or 99% identity with the amino acid sequence of SEQ ID NO: 34, or consists of SEQ ID NO: 34;
2) the heavy chain comprises the amino acid sequence as set forth in SEQ ID NO: 35 or a sequence comprising one or more amino acid substitutions (*e.g*., conservative substitutions), deletions or insertions, or any combination thereof, relative to SEQ ID NO: 35, or an amino acid sequence having at least 90%, 91%, 92%, 93%, 94%, 95%, 96%, 97%, 98%, or 99% identity with the amino acid sequence of SEQ ID NO: 35, or consists of SEQ ID NO: 35; and the light chain comprises the amino acid sequence as set forth in SEQ ID NO: 36 or a sequence comprising one or more amino acid substitutions (*e.g*., conservative substitutions), deletions or insertions, or any combination thereof, relative to SEQ ID NO: 36, or an amino acid sequence having at least 90%, 91%, 92%, 93%, 94%, 95%, 96%, 97%, 98%, or 99% identity with the amino acid sequence of SEQ ID NO: 36, or consists of SEQ ID NO: 36;
3) the heavy chain comprises the amino acid sequence as set forth in SEQ ID NO: 37 or a sequence comprising one or more amino acid substitutions (*e.g*., conservative substitutions), deletions or insertions, or any combination thereof, relative to SEQ ID NO: 37, or an amino acid sequence having at least 90%, 91%, 92%, 93%, 94%, 95%, 96%, 97%, 98%, or 99% identity with the amino acid sequence of SEQ ID NO: 37, or consists of SEQ ID NO: 37; and the light chain comprises the amino acid sequence as set forth in SEQ ID NO: 38 or a sequence comprising one or more amino acid substitutions (*e.g*., conservative substitutions), deletions or insertions, or any combination thereof, relative to SEQ ID NO: 38, or an amino acid sequence having at least 90%, 91%, 92%, 93%, 94%, 95%, 96%, 97%, 98%, or 99% identity with the amino acid sequence of SEQ ID NO: 38, or consists of SEQ ID NO: 38;
4) the heavy chain comprises the amino acid sequence as set forth in SEQ ID NO: 37 or a sequence comprising one or more amino acid substitutions (*e.g*., conservative substitutions), deletions or insertions, or any combination thereof, relative to SEQ ID NO: 37, or an amino acid sequence having at least 90%, 91%, 92%, 93%, 94%, 95%, 96%, 97%, 98%, or 99% identity with the amino acid sequence of SEQ ID NO: 37, or consists of SEQ ID NO: 37; and the light chain comprises the amino acid sequence as set forth in SEQ ID NO: 39 or a sequence comprising one or more amino acid substitutions (*e.g*., conservative substitutions), deletions or insertions, or any combination thereof, relative to SEQ ID NO: 39, or an amino acid sequence having at least 90%, 91%, 92%, 93%, 94%, 95%, 96%, 97%, 98%, or 99% identity with the amino acid sequence of SEQ ID NO: 39, or consists of SEQ ID NO: 39;
5) the heavy chain comprises the amino acid sequence as set forth in SEQ ID NO: 37 or a sequence comprising one or more amino acid substitutions (*e.g*., conservative substitutions), deletions or insertions, or any combination thereof, relative to SEQ ID NO: 37, or an amino acid sequence having at least 90%, 91%, 92%, 93%, 94%, 95%, 96%, 97%, 98%, or 99% identity with the amino acid sequence of SEQ ID NO: 37, or consists of SEQ ID NO: 37; and the light chain comprises the amino acid sequence as set forth in SEQ ID NO: 40 or a sequence comprising one or more amino acid substitutions (*e.g*., conservative substitutions), deletions or insertions, or any combination thereof, relative to SEQ ID NO: 40, or an amino acid sequence having at least 90%, 91%, 92%, 93%, 94%, 95%, 96%, 97%, 98%, or 99% identity with the amino acid sequence of SEQ ID NO: 40, or consists of SEQ ID NO: 40;
6) the heavy chain comprises the amino acid sequence as set forth in SEQ ID NO: 37 or a sequence comprising one or more amino acid substitutions (*e.g*., conservative substitutions), deletions or insertions, or any combination thereof, relative to SEQ ID NO: 37, or an amino acid sequence having at least 90%, 91%, 92%, 93%, 94%, 95%, 96%, 97%, 98%, or 99% identity with the amino acid sequence of SEQ ID NO: 37, or consists of SEQ ID NO: 37; and the light chain comprises the amino acid sequence as set forth in SEQ ID NO: 41 or a sequence comprising one or more amino acid substitutions (*e.g*., conservative substitutions), deletions or insertions, or any combination thereof, relative to SEQ ID NO: 41, or an amino acid sequence having at least 90%, 91%, 92%, 93%, 94%, 95%, 96%, 97%, 98%, or 99% identity with the amino acid sequence of SEQ ID NO: 41, or consists of SEQ ID NO: 41;
7) the heavy chain comprises the amino acid sequence as set forth in SEQ ID NO: 42 or a sequence comprising one or more amino acid substitutions (*e.g*., conservative substitutions), deletions or insertions, or any combination thereof, relative to SEQ ID NO: 42, or an amino acid sequence having at least 90%, 91%, 92%, 93%, 94%, 95%, 96%, 97%, 98%, or 99% identity with the amino acid sequence of SEQ ID NO: 42, or consists of SEQ ID NO: 42; and the light chain comprises the amino acid sequence as set forth in SEQ ID NO: 38 or a sequence comprising one or more amino acid substitutions (*e.g*., conservative substitutions), deletions or insertions, or any combination thereof, relative to SEQ ID NO: 38, or an amino acid sequence having at least 90%, 91%, 92%, 93%, 94%, 95%, 96%, 97%, 98%, or 99% identity with the amino acid sequence of SEQ ID NO: 38, or consists of SEQ ID NO: 38;
8) the heavy chain comprises the amino acid sequence as set forth in SEQ ID NO: 42 or a sequence comprising one or more amino acid substitutions (*e.g*., conservative substitutions), deletions or insertions, or any combination thereof, relative to SEQ ID NO: 42, or an amino acid sequence having at least 90%, 91%, 92%, 93%, 94%, 95%, 96%, 97%, 98%, or 99% identity with the amino acid sequence of SEQ ID NO: 42, or consists of SEQ ID NO: 42; and the light chain comprises the amino acid sequence as set forth in SEQ ID NO: 39 or a sequence comprising one or more amino acid substitutions (*e.g*., conservative substitutions), deletions or insertions, or any combination thereof, relative to SEQ ID NO: 39, or an amino acid sequence having at least 90%, 91%, 92%, 93%, 94%, 95%, 96%, 97%, 98%, or 99% identity with the amino acid sequence of SEQ ID NO: 39, or consists of SEQ ID NO: 39;
9) the heavy chain comprises the amino acid sequence as set forth in SEQ ID NO: 42 or a sequence comprising one or more amino acid substitutions (*e.g*., conservative substitutions), deletions or insertions, or any combination thereof, relative to SEQ ID NO: 42, or an amino acid sequence having at least 90%, 91%, 92%, 93%, 94%, 95%, 96%, 97%, 98%, or 99% identity with the amino acid sequence of SEQ ID NO: 42, or consists of SEQ ID NO: 42; and the light chain comprises the amino acid sequence as set forth in SEQ ID NO: 40 or a sequence comprising one or more amino acid substitutions (*e.g*., conservative substitutions), deletions or insertions, or any combination thereof, relative to SEQ ID NO: 40, or an amino acid sequence having at least 90%, 91%, 92%, 93%, 94%, 95%, 96%, 97%, 98%, or 99% identity with the amino acid sequence of SEQ ID NO: 40, or consists of SEQ ID NO: 40;
10) the heavy chain comprises the amino acid sequence as set forth in SEQ ID NO: 42 or a sequence comprising one or more amino acid substitutions (*e.g*., conservative substitutions), deletions or insertions, or any combination thereof, relative to SEQ ID NO: 42, or an amino acid sequence having at least 90%, 91%, 92%, 93%, 94%, 95%, 96%, 97%, 98%, or 99% identity with the amino acid sequence of SEQ ID NO: 42, or consists of SEQ ID NO: 42; and the light chain comprises the amino acid sequence as set forth in SEQ ID NO: 41 or a sequence comprising one or more amino acid substitutions (*e.g*., conservative substitutions), deletions or insertions, or any combination thereof, relative to SEQ ID NO: 41, or an amino acid sequence having at least 90%, 91%, 92%, 93%, 94%, 95%, 96%, 97%, 98%, or 99% identity with the amino acid sequence of SEQ ID NO: 41, or consists of SEQ ID NO: 41;
11) the heavy chain comprises the amino acid sequence as set forth in SEQ ID NO: 43 or a sequence comprising one or more amino acid substitutions (*e.g*., conservative substitutions), deletions or insertions, or any combination thereof, relative to SEQ ID NO: 43, or an amino acid sequence having at least 90%, 91%, 92%, 93%, 94%, 95%, 96%, 97%, 98%, or 99% identity with the amino acid sequence of SEQ ID NO: 43, or consists of SEQ ID NO: 43; and the light chain comprises the amino acid sequence as set forth in SEQ ID NO: 38 or a sequence comprising one or more amino acid substitutions (*e.g*., conservative substitutions), deletions or insertions, or any combination thereof, relative to SEQ ID NO: 38, or an amino acid sequence having at least 90%, 91%, 92%, 93%, 94%, 95%, 96%, 97%, 98%, or 99% identity with the amino acid sequence of SEQ ID NO: 38, or consists of SEQ ID NO: 38;
12) the heavy chain comprises the amino acid sequence as set forth in SEQ ID NO: 43 or a sequence comprising one or more amino acid substitutions (*e.g*., conservative substitutions), deletions or insertions, or any combination thereof, relative to SEQ ID NO: 43, or an amino acid sequence having at least 90%, 91%, 92%, 93%, 94%, 95%, 96%, 97%, 98%, or 99% identity with the amino acid sequence of SEQ ID NO: 43, or consists of SEQ ID NO: 43; and the light chain comprises the amino acid sequence as set forth in SEQ ID NO: 39 or a sequence comprising one or more amino acid substitutions (*e.g*., conservative substitutions), deletions or insertions, or any combination thereof, relative to SEQ ID NO: 39, or an amino acid sequence having at least 90%, 91%, 92%, 93%, 94%, 95%, 96%, 97%, 98%, or 99% identity with the amino acid sequence of SEQ ID NO: 39, or consists of SEQ ID NO: 39;
13) the heavy chain comprises the amino acid sequence as set forth in SEQ ID NO: 43 or a sequence comprising one or more amino acid substitutions (*e.g*., conservative substitutions), deletions or insertions, or any combination thereof, relative to SEQ ID NO: 43, or an amino acid sequence having at least 90%, 91%, 92%, 93%, 94%, 95%, 96%, 97%, 98%, or 99% identity with the amino acid sequence of SEQ ID NO: 43, or consists of SEQ ID NO: 43; and the light chain comprises the amino acid sequence as set forth in SEQ ID NO: 40 or a sequence comprising one or more amino acid substitutions (*e.g*., conservative substitutions), deletions or insertions, or any combination thereof, relative to SEQ ID NO: 40, or an amino acid sequence having at least 90%, 91%, 92%, 93%, 94%, 95%, 96%, 97%, 98%, or 99% identity with the amino acid sequence of SEQ ID NO: 40, or consists of SEQ ID NO: 40;
14) the heavy chain comprises the amino acid sequence as set forth in SEQ ID NO: 43 or a sequence comprising one or more amino acid substitutions (*e.g*., conservative substitutions), deletions or insertions, or any combination thereof, relative to SEQ ID NO: 43, or an amino acid sequence having at least 90%, 91%, 92%, 93%, 94%, 95%, 96%, 97%, 98%, or 99% identity with the amino acid sequence of SEQ ID NO: 43, or consists of SEQ ID NO: 43; and the light chain comprises the amino acid sequence as set forth in SEQ ID NO: 41 or a sequence comprising one or more amino acid substitutions (*e.g*., conservative substitutions), deletions or insertions, or any combination thereof, relative to SEQ ID NO: 41, or an amino acid sequence having at least 90%, 91%, 92%, 93%, 94%, 95%, 96%, 97%, 98%, or 99% identity with the amino acid sequence of SEQ ID NO: 41, or consists of SEQ ID NO: 41.

In certain embodiments of the antibody of any one of the preceding items, the antibody is monoclonal. In a preferred embodiment, the antibody is a chimeric antibody, in another more preferred embodiment, the antibody is a humanized antibody.

In certain embodiments of the antibody of any one of the preceding items, the antibody is a full length antibody.

In one embodiment, the anti-BDCA-2 antibody of the present disclosure is an intact antibody, *e.g*., an IgG1, IgG2, IgG3, IgG4 antibody. In another embodiment, the anti-BDCA-2 antibody of the present disclosure encompasses only antigen binding portions thereof, such as: fab, Fab '-SH, Fv, scFv or (Fab') ₂ fragments.

In a second aspect, the present disclosure provides an isolated polynucleotide molecule encoding any one of the antibodies or antigen-binding fragments thereof of the first aspect.

In a third aspect, the present disclosure provides a vector comprising the nucleic acid molecule of the second aspect. In one embodiment, the vector is an expression vector.

In a fourth aspect, the present disclosure provides a host cell comprising the vector of the third aspect or the nucleic acid molecule of the second aspect. In some embodiments, the host cell is prokaryotic, such as *e.g.*, *E. coli.* In other embodiments, the host cell is eukaryotic, such as a 293 cell, CHO cell, yeast cell, or plant cell.

In a fifth aspect, the present disclosure provides a conjugate comprising the antibody or antigen-binding fragment thereof of the present disclosure and a conjugating moiety, the conjugating moiety being another molecule; preferably, the conjugating moiety is a radioisotope, a fluorescent substance, a luminescent substance, a colored substance, an enzyme, or the like.

In a sixth aspect, the present disclosure provides a pharmaceutical composition comprising the antibody or antigen-binding fragment thereof of the present disclosure or the polynucleotide molecule, vector, host cell, conjugate of the present disclosure and a pharmaceutically acceptable carrier.

In a seventh aspect, the present disclosure provides a method of making the anti-BDCA-2 antibody or antigen-binding fragment thereof described herein, the method including expressing the antibody or antigen-binding fragment thereof in the host cell described herein under conditions suitable for expression of the antibody or antigen-binding fragment thereof, and recovering the expressed antibody or antigen-binding fragment thereof from the host cell.

In an eighth aspect, the present disclosure provides a method of treating a disease in a subject in need thereof, including administering to the subject a therapeutically effective amount of the antibody or antigen-binding fragment thereof of the present disclosure or the polynucleotide molecule, vector, host cell, conjugate of the present disclosure, or a therapeutically effective amount of the pharmaceutical composition of the present disclosure.

In one embodiment, the disease is an autoimmune disease or cancer, such as Systemic Lupus Erythematosus (SLE), scleroderma, polymyositis, dermatomyositis, psoriasis, Sjogren's syndrome, rheumatoid arthritis, Graves' disease, and Hashimoto's disease; breast tumors, ovarian cancer, Multiple Myeloma (MM), and the like.

In a ninth aspect, the present disclosure provides a method of preventing and/or treating a tumor or an autoimmune disease in a subject, the method including administering to the subject in need thereof an effective amount of the antibody or antigen-binding fragment thereof of the present disclosure or the polynucleotide molecule, vector, host cell, conjugate of the present disclosure, or the pharmaceutical composition of the present disclosure, or the like.

In one embodiment, the present disclosure provides the antibody or antigen-binding fragment thereof disclosed herein or the polynucleotide molecule, vector, host cell, conjugate or pharmaceutical composition of the present disclosure for use as a medicament.

In one embodiment, the present disclosure provides the antibody or antigen-binding fragment thereof disclosed herein or the polynucleotide molecule, vector, host cell, conjugate or pharmaceutical composition of the present disclosure for use in therapy.

In a specific embodiment, the present disclosure provides a method of reducing, eliminating pDC cells in circulation, including administering to the subject an effective amount of the antibody or antigen-binding fragment thereof of the present disclosure, or the polynucleotide molecule, vector, host cell, conjugate of the present disclosure, or an effective amount of the pharmaceutical composition of the present disclosure.

In a tenth aspect, the present disclosure provides use of the anti-BDCA-2 antibody or antigen-binding fragment thereof, or the polynucleotide molecule, vector, host cell, conjugate or pharmaceutical composition of the present disclosure in the manufacture of a medicament for the treatment of an autoimmune disease or cancer.

In one embodiment, any of the antibodies or antigen-binding fragments thereof of the first aspect are used to treat an autoimmune disease or cancer. In one embodiment, the autoimmune disease or cancer is, for example, autoimmune disease such as Systemic Lupus Erythematosus (SLE), scleroderma, polymyositis, dermatomyositis, psoriasis, Sjogren's syndrome, rheumatoid arthritis, Graves' disease, and Hashimoto's disease; breast tumor, ovarian cancer, Multiple Myeloma (MM), and the like.

In an eleventh aspect, the present disclosure provides a kit comprising the antibody or antigen-binding fragment or conjugate thereof of the present disclosure.

In a twelfth aspect, the present disclosure provides a method of detecting the presence or level of BDCA-2 in a sample including the steps of contacting the antibody or antigen-binding fragment thereof or conjugate of the present disclosure with the sample and detecting whether the antibody or antigen-binding fragment thereof or conjugate forms a complex with BDCA-2.

The present disclosure is further illustrated in the following figures and detailed description. However, these drawings and specific embodiments should not be construed as limiting the scope of the present disclosure, and modifications readily contemplated to those skilled in the art are intended to be included within the spirit of the present application and within the scope of the appended claims.

### DESCRIPTION OF DRAWINGS

FIG. 1 shows the binding activity of purified hybridoma supernatant to Jurkat-hBDCA-2 cells, with BIIB059 as the control antibody. FIG. 1A shows the binding activity of hybridoma 17G6A2 supernatant to jurkat-hBDCA-2 cells, FIG. 1B shows the binding activity of hybridoma 164C11B8 supernatant to jurkat-hBDCA-2 cells, FIG. 1C shows the binding activity of hybridoma 17G6A2 supernatant to jurkat-cynoBDCA-2 cells, and FIG. 1D shows the binding activity of hybridoma 164C11B8 supernatant to jurkat-cynoBDCA-2 cells.
FIG. 2 shows the inhibitory activity of purified hybridoma supernatant against CpGA-induced type I interferon production by human PBMC cells, FIG. 2A shows the activity of hybridoma 17G6A2, FIG. 2B shows the activity of hybridoma 164C11B8, and BIIB059 is the control antibody.
FIG. 3A shows the binding activity of chimeric antibodies to jurkat h-BDCA-2 cells, FIG. 3B shows the binding activity of chimeric antibodies to jurkat-cyno-BDCA-2 cells, and BIIB059 is the control antibody.
FIG. 4 shows the inhibitory activity of chimeric antibodies against CpGA-induced type I interferon production by human PBMC cells, FIG. 4A shows the activity of chimeric antibody 17G6A2_ hIgG1, FIG. 4B shows the activity of chimeric antibody 164C11B8_ hIgG1, and BIIB059 is the control antibody.
FIG. 5 shows the inhibitory activity of chimeric antibodies against immune complexes-induced type I interferon production in human pDC cells, FIG. 5A shows the activity of chimeric antibody 17G6A2_ hIgG1, FIG. 5B shows the activity of chimeric antibody 164C11B8_ hIgG1, and BIIB059 is the control antibody.
FIG. 6 shows the activity of chimeric antibodies in activating Jurkat-human FcγR IIIa (158V) -NFAT cells.

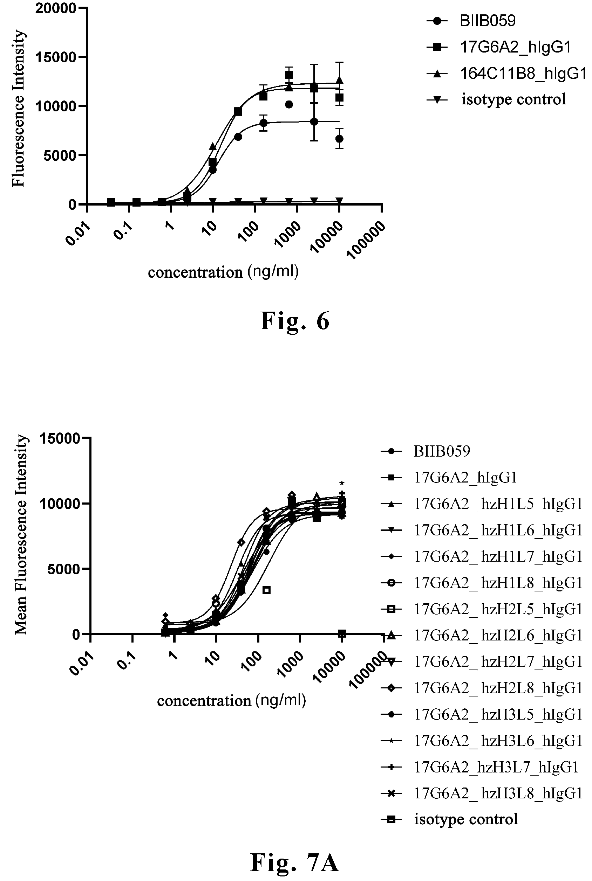

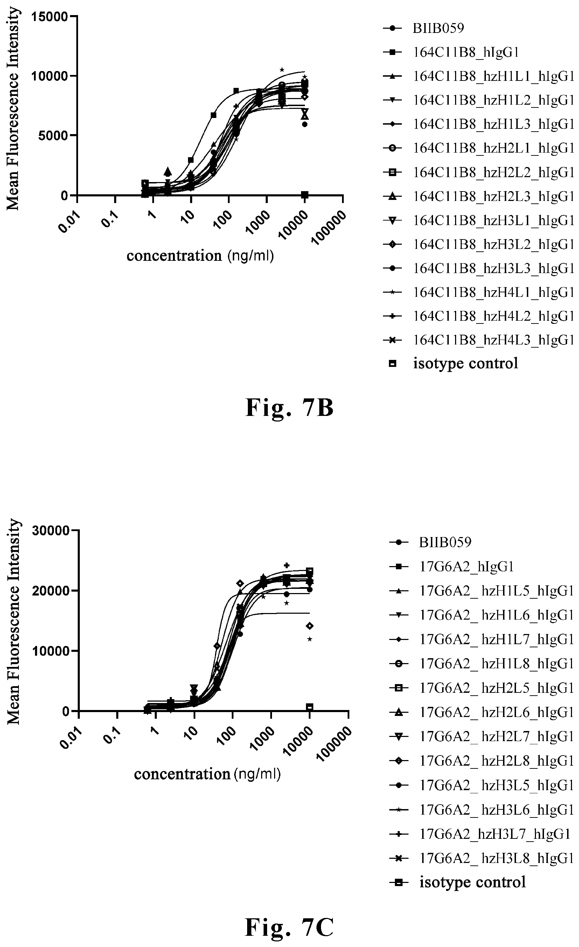

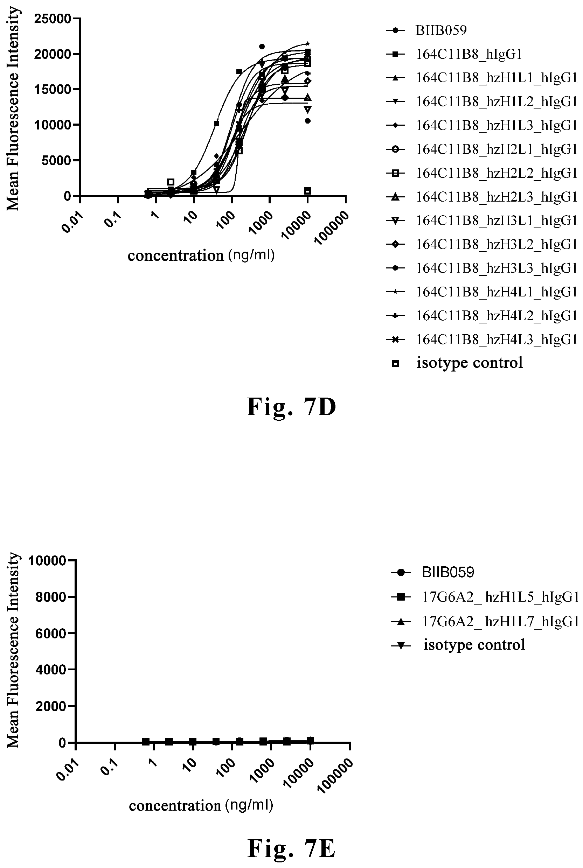

FIG. 7 shows the binding activity of humanized antibodies to jurkat cells expressing BDCA-2, FIG. 7A shows the activity of the humanized antibody of 17G6A2 on jurkat cells expressing human BDCA-2, FIG. 7B shows the activity of the humanized antibody of 164C11B8 on jurkat cells expressing human BDCA-2, FIG. 7C shows the activity of the humanized antibody of 17G6A2 on jurkat cells expressing cynomolgus monkey BDCA-2, FIG. 7D shows the activity of the humanized antibody of 164C11B8 on jurkat cells expressing cynomolgus monkey BDCA-2, FIG. 7E shows that neither the humanized antibodies of 17G6A2 nor BIIB059 bind to jurkat blank cells.
FIG. 8 shows the inhibitory activity of the humanized antibody of 17G6A2 on CpGA-induced type I interferon production by human PBMC cells
FIG. 9 shows the inhibitory activity of the humanized antibody of 17G6A2 on immune complexes-induced type I interferon production in human pDC cells.
FIG. 10 shows the binding activity of humanized antibodies to human pDC.
FIG. 11A shows that the anti-BDCA-2 antibody does not bind to CLEC4A, FIG. 11B shows that the anti-BDCA-2 antibody does not bind to CLEC4D, FIG. 11C shows that the anti-BDCA-2 antibody does not bind to CLEC6A, and FIG. 11D shows that the anti-BDCA-2 antibody does not bind to CLEC4E.
FIG. 12 shows the activity of humanized antibodies in activating Jurkat-human FcyR IIIa (158V) -NFAT cells.
FIG. 13 shows the induction of BDCA2 internalization on the surface of pDC cells by 17G6A2_hzH1L5_hIgG1 or BIIB059.
FIG. 14 shows the binding of 17G6A2_hzH1L5_hIgG1 or BIIB059 to BDCA2 on the surface of cynomolgus monkey pDC cells.

### DETAILED DESCRIPTION

All publications, patent applications, patents, and other references mentioned herein are incorporated by reference in their entirety. In addition, the materials, methods, and examples described herein are illustrative only and are not intended to be limiting. Other features, objects, and advantages of the present disclosure will be apparent from the description and drawings, and from the claims. Unless defined otherwise, all technical and scientific terms used herein have the same meaning as commonly understood by one of ordinary skill in the art. For the purposes of the present disclosure, the following terms are defined below.

### Definition

The term "about" when used in conjunction with a numerical value is intended to encompass the numerical value within a range having a lower limit that is 5% less than the stated numerical value and an upper limit that is 5% greater than the stated numerical value.

The term "and/or" should be understood to mean any one of the options or a combination of any two or more of the options.

As used herein, the term "comprising" or "comprises" is intended to mean that the stated element, integer or step is included, but does not exclude any other element, integer or step. When the term "comprising" or " comprises" is used herein, unless otherwise indicated, it also encompasses the situation consisting of the stated elements, integers or steps.

The term "antibody" is used herein in the broadest sense and refers to a protein that comprises an antigen binding site.

The term "immunoglobulin" refers to a protein having the structure of a naturally occurring antibody, and is often used interchangeably with the term "antibody" in the present application. Immunoglobulins of the IgG class are heterotetrameric glycoproteins composed of two light chains and two heavy chains that are disulfide-bonded. From N-terminus to C-terminus, each immunoglobulin heavy chain has one heavy chain variable region (VH), also known as a heavy chain variable domain, followed by three heavy chain constant domains (CH1, CH2, and CH 3). Similarly, from N-terminus to C-terminus, each immunoglobulin light chain has a light chain variable region (VL), also known as a light chain variable domain, followed by a light chain constant domain (CL). In IgG molecules, usually the VH-CH1 of the heavy chain pairs with the VL-CL of the light chain to form a Fab fragment that specifically binds to the antigen. Thus, an IgG immunoglobulin essentially consists of two Fab molecules connected by an immunoglobulin hinge region and two dimerized Fc regions. Heavy chains of immunoglobulins can be assigned to one of 5 classes, called α (IgA), δ (IgD), ε (IgE),γ(IgG), or µ (IgM), based on the type of their constant region, with some classes being further divided into subclasses, such as γ1(IgG1), γ2(IgG2), γ3(IgG3), γ4(IgG4), α 1(IgA1), and α 2(IgA 2). Immunoglobulin light chains can also be divided into one of two types, called kappa and lambda, based on the amino acid sequence of their constant domains.

"Complementarity determining regions" or "CDR regions" or "CDRs" or "hypervariable regions" are regions of an antibody variable domain which are highly variable in sequence and form structurally defined loops ("hypervariable loops") and/or contain antigen-contacting residues ("antigen-contacting points"). The CDRs are primarily responsible for binding to an antigenic epitope.

Various schemes for determining the CDR sequences of a given VH or VL amino acid sequence are known in the art: the Kabat Complementarity Determining Regions (CDRs) are determined based on sequence variability and are most commonly used (Kabat et al., Sequences of Proteins of Immunological Interest, 5 th edition, Public Health Service, National Institutes of Health, Bethesda, Md. (1991)), while Chothia refers to the position of the structural loops (Chothia et al., (1987) J. mol. biol. 196: 901) 917; Chothia et al. (1989) Nature 342: 877-883. AbM CDR is compromised between Kabat CDR and Chothia structural ring, and is used by AbM Antibody Modeling Software, Oxford Molecular. "Contact" CDR is based on the analysis of available complex crystal structures. The residues of each of these CDRs are described below according to different CDR determination schemes.

| CDR | Kabat scheme | AbM scheme | Chothia scheme | Contact scheme |
|---|---|---|---|---|
| CDR1 | L24-L34 | L24-L34 | L26-L32 | L30-L36 |
| CDR2 | L50-L56 | L50-L56 | L50-L52 | L46-L55 |
| CDR3 | L89-L97 | L89-L97 | L91-L96 | L89-L96 |
| CDR1 | H31-H35B | H26-H35B | H26-H32 | H30-H35B |
| (Kabat numbering system) | | | | |
| CDR1 | H31-H35 | H26-H35 | H26-H32 | H30-H35 |
| (Chothia numbering system) | | | | |
| CDR2 | H50-H65 | H50-H58 | H53-H55 | H47-H58 |
| CDR3 | H95-H102 | H95-H102 | H96-H101 | H93-H101 |
| (Kabat numbering system) | | | | |

In reference to defining an antibody with a particular CDR sequence defined herein, the scope of the antibody also encompasses antibodies whose variable region sequences comprise the particular CDR sequence but whose claimed CDR boundaries differ from the particular CDR boundaries defined herein due to the application of different schemes (*e.g*., different assignment system rules or combinations).

The CDRs of the antibodies of the present disclosure can be assessed manually to determine boundaries according to any scheme or combination thereof in the art. Unless otherwise indicated, in the present disclosure, the term "CDR" or "CDR sequence" encompasses CDR sequences determined in any of the ways described above.

The terms "antigen binding site" and "antigen binding domain" are used interchangeably to refer to the region of an antibody molecule that actually binds to an antigen. Antigen binding sites include, but are not limited to, Fv, Fab fragments, Fab', Fab'-SH, F(ab')₂, single chain antibody molecules (*e.g*., scFv), VHH, and the like.

The term "variable region" or "variable domain" of an antibody refers to a domain of an antibody heavy or light chain that is involved in binding of the antibody to an antigen. The variable region of an antibody can be further subdivided into hypervariable regions (*i.e.*, Complementarity Determining Regions (CDRs)) and more conserved regions (*i.e.*, Framework Regions (FRs)) interspersed between the hypervariable regions. In the case of IgG type immunoglobulins, the heavy or light chain variable region comprises, in order from N-terminus to C-terminus, FR1-CDR1-FR2-CDR2-FR3-CDR3-FR4. In the case of heavy chain antibodies (also referred to herein as nanobodies), such as those from the camelid family, the antigen-binding site consists of a single VH domain (*i.e.*, a "VHH" domain). The VHH of a native heavy chain antibody has a similar structure as the heavy chain variable region of a native IgG antibody, *i.e*. it comprises four conserved Framework Regions (FRs) and three Complementarity Determining Regions (CDRs).

"Heavy chain constant region domain" or "heavy chain constant region" refers to a constant region domain from or obtained from or derived from an immunoglobulin heavy chain, comprising the heavy chain constant regions CH1, CH2, CH3, and optionally the heavy chain constant region CH4, which are covalently linked sequentially from N-terminus to C-terminus. In most cases, the heavy chain constant regions CH1 and CH2 are connected by a heavy chain hinge region, but may also be connected by a flexible linker, where appropriate.

The term "EC₅₀ ," also known as "half effective concentration," refers to the concentration of a drug, antibody or toxin that induces a response that is 50% between baseline and maximum after a specified exposure time.

As used herein, the term "binding" or "specific binding" means that the binding is selective for the antigen and can be distinguished from unwanted or non-specific interactions. The ability of an antigen binding site to bind to a particular antigen can be determined by enzyme-linked immunosorbent assay (ELISA) or conventional binding assays known in the art.

"Affinity" or "binding affinity" refers to the intrinsic binding capacity reflecting the interaction between members of a binding pair. The affinity of molecule X for its binding partner Y may be represented by the equilibrium dissociation constant (K_{D}), which is the ratio of the dissociation and association rate constants (k_{dis} and kₒₙ, respectively). Binding affinity can be measured by common methods known in the art.

The amino acid mutation may be an amino acid substitution, deletion, insertion and/or addition. In some embodiments, an amino acid mutation is a substitution of one or more amino acids, such as a single amino acid substitution or a combination of multiple amino acid substitutions. Amino acid deletions and insertions include those occurring at the amino and/or carboxy terminus of the polypeptide sequence, as well as those occurring within the polypeptide sequence. Amino acid substitutions of the present disclosure optionally include conservative substitutions of amino acids.

"Percentage (%) identity" of an amino acid sequence refers to the percentage of amino acid residues in the candidate sequence that are identical to the amino acid residues in the specific amino acid sequence shown in this specification, after aligning the candidate sequence with the specific amino acid sequence shown in this specification and introducing gaps, if necessary, to achieve the maximum percentage of sequence identity, and not considering any conservative substitutions as part of the sequence identity. In some embodiments, the present disclosure contemplates variants of the antibody molecules of the present disclosure having a substantial degree of identity, such as at least 80%, 85%, 90%, 95%, 97%, 98% or 99% or more, relative to the antibody molecules and sequences thereof specifically disclosed herein. The variant may contain conservative modifications.

For polypeptide sequences, "conservative modifications" include substitutions, deletions, or additions to the polypeptide sequence that result in the replacement of one amino acid with a chemically similar amino acid. Tables providing conservative substitutions of functionally similar amino acids are well known in the art. These types of conservative modifications are in addition to, and do not exclude, the polymorphic variants, interspecies homologs, and alleles of the present disclosure. The following eight groups contain amino acids that are conservative substitutions for each other: 1) alanine (A), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M) (see, *e.g*., Creighton, Proteins (1984)). In some embodiments, the term "conservative sequence modifications" is used to refer to amino acid modifications that do not significantly affect or alter the binding characteristics of the antibody containing the amino acid sequence.

The terms "individual" or "subject" are used interchangeably and refer to a mammal. Mammals include, but are not limited to, domesticated animals (*e.g*., cows, sheep, cats, dogs, and horses), primates (*e.g*., human and non-human primates such as monkeys), rabbits and rodents (*e.g*., mice and rats). In particular, the individual is a human.

The term "treatment" refers to clinical intervention intended to alter the natural course of disease in the individual undergoing treatment. Desirable therapeutic effects include, but are not limited to, preventing the occurrence or recurrence of disease, alleviating symptoms, reducing any direct or indirect pathological consequences of the disease, preventing metastasis, reducing the rate of disease progression, improving or palliating the disease state, and alleviating or improving prognosis. In some embodiments, the antibody molecules of the present disclosure are used to delay the progression of a disease or to slow the progression of a disease.

The term "prevention" includes inhibition of the onset or progression of a disease or disorder or the symptoms of a particular disease or disorder. In some embodiments, subjects with a family history of cancer are candidates for a prophylactic regimen. Generally, in the context of cancer, the term "prevention" refers to the administration of a drug prior to the onset of signs or symptoms of cancer, particularly in a subject at risk for cancer.

The term "effective amount" refers to an amount or dose of an antibody or composition of the present disclosure that, upon administration to a patient in a single or multiple doses, produces the desired effect in the patient in need of treatment or prevention. An effective amount can be readily determined by the attending physician, as one skilled in the art, by considering a number of factors including: a species such as a mammal; body weight, age, and general health; the specific diseases involved; the degree or severity of the disease; the response of the individual patient; the specific antibody administered; a mode of administration; bioavailability characteristics of the administered preparation; a selected dosing regimen; and the use of any concomitant therapies.

The term "therapeutically effective amount" refers to an amount effective, at dosages and for periods of time required, to achieve the desired therapeutic result. A therapeutically effective amount of an antibody or antibody fragment or composition may vary according to factors such as the disease state, the age, sex, and weight of the individual, and the ability of the antibody or antibody portion to elicit a desired response in the individual. A therapeutically effective amount is also a dose in which any toxic or detrimental effects of the antibody or antibody fragment or composition are less than the therapeutically beneficial effects. A "therapeutically effective amount" preferably inhibits a measurable parameter by at least about 20%, more preferably by at least about 40%, even more preferably by at least about 50%, 60% or 70%, and still more preferably by at least about 80% or 90%, relative to an untreated subject.

The term "prophylactically effective amount" refers to an amount effective, at dosages and for periods of time required, to achieve the desired prophylactic result. Typically, since a prophylactic dose is used in a subject prior to or at an earlier stage of disease, the prophylactically effective amount will be less than the therapeutically effective amount.

The term "pharmaceutical composition" refers to a composition that is present in a form that allows for the biological activity of the active ingredients contained therein to be effective, and that does not contain additional ingredients that have unacceptable toxicity to a subject to whom the composition is administered.

### Polynucleotides, vectors and hosts

The present disclosure provides nucleic acids encoding any of the above antibody molecules or antigen-binding fragments thereof. Polynucleotide sequences encoding the antibody molecules or antigen-binding fragments thereof of the present disclosure can be generated by de novo solid phase DNA synthesis or by genetic engineering methods using methods well known in the art. Furthermore, the polynucleotides and nucleic acids of the present disclosure may include segments encoding secretion signal peptides and be operatively linked to segments encoding antibody molecules of the present disclosure or antigen-binding fragments thereof, thereby guiding the secretory expression of antibody molecules of the present disclosure or antigen-binding fragments thereof.

The present disclosure also provides a vector comprising the nucleic acid of the present disclosure. In one embodiment, the vector is an expression vector, such as eukaryotic and prokaryotic expression vectors. "Expression vector" refers to a vector comprising a recombinant polynucleotide comprising an expression control sequence operably linked to a nucleotide sequence to be expressed. The expression vector contains sufficient cis-acting elements for expression; other elements for expression may be provided by the host cell or in an *in vitro* expression system. Expression vectors include all those known in the art, including cosmids, plasmids (*e.g*., naked or contained in liposomes) and viruses (*e.g*., lentiviruses, retroviruses, adenoviruses, and adeno-associated viruses) into which the recombinant polynucleotide is incorporated.

The present disclosure also provides prokaryotic and eukaryotic host cells comprising said nucleic acid or said vector. Host cells include "transformants" and "transformed cells," which include the primary transformed cell and progeny derived therefrom. The host cell is any type of cell system that can be used to produce the antibody molecule of the present disclosure, including eukaryotic cells, *e.g.*, mammalian cells, insect cells, yeast cells; and prokaryotic cells, *e.g.*, E.coli cells. Host cells include cultured cells, and also include cells within transgenic animals, transgenic plants, or cultured plant tissues or animal tissues. Host cells suitable for replicating and supporting the expression of the antibody molecules or antigen-binding fragments thereof of the present disclosure are well known in the art. Such cells can be transfected or transduced with a particular expression vector, and large numbers of vector-containing cells can be grown for inoculation of large-scale fermenters, thereby obtaining sufficient quantities of the antibody molecules.

### Pharmaceutical composition and pharmaceutical preparation

The present disclosure also includes compositions (including pharmaceutical compositions or pharmaceutical preparations) comprising the antibody molecules or antigen-binding fragments thereof of the present disclosure and compositions comprising polynucleotides encoding the antibody molecules or antigen-binding fragments thereof of the present disclosure. These compositions may also optionally contain suitable pharmaceutical auxiliary materials such as pharmaceutical carriers, pharmaceutical excipients known in the art, including buffers.

The pharmaceutical compositions or preparations of the present disclosure may also comprise or be used in combination with one or more other active ingredients. The active ingredients are required for the particular indication being treated, preferably those having complementary activities that do not adversely affect each other. For example, it would be desirable to also provide other active ingredients for the treatment of an autoimmune disease, which are suitably present in combination in amounts effective for the intended use.

### Methods of treatment and uses

The terms "individual" or "subject" are used interchangeably herein and refer to a mammal. Mammals include, but are not limited to, domesticated animals (*e.g*., cows, sheep, cats, dogs, and horses), primates (*e.g*., human and non-human primates such as monkeys), rabbits and rodents (*e.g*., mice and rats). In particular, the subject is human.

The present disclosure provides a method of treating an autoimmune disease or cancer in a subject in need thereof, including administering to the subject a therapeutically effective amount of the antibody or antigen-binding fragment thereof of the present disclosure, or a therapeutically effective amount of the pharmaceutical composition of the present disclosure. In one embodiment, the autoimmune disease or cancer is, for example, autoimmune disease such as Systemic Lupus Erythematosus (SLE), scleroderma, polymyositis, dermatomyositis, psoriasis, Sjoegren's syndrome, rheumatoid arthritis, Graves' disease, and hashimoto's disease; breast tumors, ovarian cancer, Multiple Myeloma (MM), and the like.

In one embodiment, the present disclosure provides a method of reducing, depleting pDC cells expressing BDCA-2 in the circulation, including administering to the subject an effective amount of the antibody or antigen-binding fragment thereof of the present disclosure, or an effective amount of the pharmaceutical composition of the present disclosure.

The antibody molecules or antigen-binding fragments thereof of the present disclosure (and pharmaceutical compositions comprising the same) may be administered by any suitable method, including parenteral administration, intrapulmonary administration and intranasal administration, and, if local treatment is required, intralesional administration. Parenteral infusion includes intramuscular, intravenous, intraarterial, intraperitoneal or subcutaneous administration. Administration may be by any suitable route, *e.g.*, by injection, *e.g.*, intravenous or subcutaneous injection, depending in part on whether administration is short-term or long-term. Various dosing schedules are contemplated herein, including, but not limited to, a single administration or multiple administrations at multiple time points, bolus administration, and pulsed infusion.

For the prevention or treatment of a disease, the appropriate dosage of the antibody molecule or antigen-binding fragment thereof of the present disclosure (when used alone or in combination with one or more other therapeutic agents) will depend on the type of disease to be treated, the severity and course of the disease, whether administered for prophylactic or therapeutic purposes, previous therapy, the patient's clinical history, and the discretion of the attending physician.

The following examples are described to aid in the understanding of the present disclosure. The examples are not intended to, and should not be construed as, limiting the scope of the present disclosure in any way.

### EXAMPLES

The experimental methods described in the following examples are, unless otherwise specified, all conventional methods in the art using default parameters, procedures, and the like; the experimental materials used, unless otherwise specified, are all commercially available products. In the examples where specific techniques or conditions are not described, the procedures follow those disclosed in the literature in the relevant field or are conducted according to the instructions provided in the corresponding product manuals. Reagents or instruments without specified manufacturers are all conventional products that are commercially available through standard channels.

### Example 1: Mouse hybridomas producing anti-human BDCA-2

This example immunizes mice in two ways to obtain antibodies against human BDCA-2. That is, mice were immunized with mRNA (manufactured by Shanghai Hongcheng Pharmaceutical Co. Ltd.) encoding full-length human BDCA-2 (UniProtKB: Q8WTT0) and extracellular fragments of human BDCA-2 (UniProtKB: Q8WTT0) protein, respectively, as immunogens. The specific method is as follows:

### 1. Animal immunization

A. The extracellular fragment of human BDCA-2 protein was used as an immunogen and mixed 1:1 with Freund's adjuvant (SIGMA, cat# F5506 and F5881). The protein was then administered to 6-8 week old female Balb/c mice and female SJL mice using standard methods. After the first immunization of 50 µg/mouse, a second immunization of 25 µg/mouse was given after an interval of 2 weeks, followed by a third immunization of 25 µg/mouse after an interval of 2 weeks. Ten days after the last immunization, a booster immunization was given using the same protein fragment. Three days later, the spleen of the mice was harvested for cell fusion.

B. The mRNA encoding full-length human BDCA-2 was used as an immunogen (50 µg/mouse), packaged in lipid nanoparticles (LNP), and then used to immunize female Balb/c mice and female SJL mice with the mRNA according to the standard method. mRNA immunization was repeated 3 or 4 times at 3-week intervals. Seven days after the last immunization, the same protein was used for booster immunization. Three days later, the spleen of the mouse was harvested for cell fusion.

### 2. Cell fusion

Mouse spleen cells were electrofused with SP2/0 cells (ATCC No. CRL-1581) at a ratio of 2:1 (BTX electrofusion apparatus: ECM2001⁺), cultured in HAT (GIBCO, cat# H0262) medium in 96-well culture plates. After 10 days, antibodies were screened from the hybridoma cell supernatant.

### 3. Screening for positive clones

Cells expressing human BDCA-2 (Jurkat-hBDCA-2, constructed by Genomeditech (Shanghai) Co., Ltd.) and cynomolgus monkey BDCA-2 (Jurkat-cynoBDCA-2, constructed by Genomeditech (Shanghai) Co., Ltd.) were separately plated in 96-well U-shaped assay plates at a density of 2 ×10⁶ /mL, 50µL /well. 50 µ L of hybridoma cell culture supernatant was added and incubated at 4 °C for 1 hour. Cells were washed and centrifugated twice with FACS buffer (1% FBS in PBS) at 300 g. Secondary AlexaFlour-647-labeled anti-mouse antibody (Invitrogen, cat # A21445) was added at 1: 1000 dilution and incubated for 30 min. Cells were washed and centrifugated 3 times with FACS buffer at 300 g. The Mean Fluorescence Intensity (median Fluorescence Intensity, MFI) of each well was read by a flow cytometer (BECKMAN COULTER cytoFLEX). Wells with a higher mean fluorescence intensity (MFI) for binding to human BDCA-2 and cynomolgus monkey BDCA-2 than the isotype control indicate that the corresponding clone cells secrete antibodies that bind to human and cynomolgus monkey BDCA-2 proteins. Cells within the positive clone culture well were gently mixed and aspirated into centrifuge tubes, an appropriate amount of culture medium was added, and after mixing, a small number of cells were aspirated for counting. Based on the counting results, the hybridoma cells were diluted to 5 cells per mL, and 0.2 mL of the above cell suspension was added to each well of a 96-well plate. After one week of culture, the culture supernatant containing a single cell colony was selected and detected again using the above method. The corresponding clones are shown in Table 1.

**Table 1. Positive hybridoma clones that bind to human BDCA-2 and cynomolgus monkey BDCA-2**

| Clone No. | MFI of binding to Jurkat-hBDCA-2 cells | MFI of binding to Jurkat-cynoBDCA-2 cells |
|---|---|---|
| 17G6A2 | 57354.9 | 154741.5 |
| 164C11B8 | 154285.8 | 391032.8 |
| Isotype control | 17.5 | 67.3 |

### Example 2: Preparation, purification and functional identification of murine monoclonal antibody against human BDCA-2

Following standard procedures, hybridoma cells that exhibited binding activity to human and cynomolgus monkey BDCA-2 as shown in Table 1 were cultured in serum-free medium. After 10 days, the culture supernatant was collected, and murine monoclonal antibodies against human BDCA-2 were purified using a Protein A column (Boglon (Shanghai) Biotechnology Co., Ltd., cat # AA0272) to obtain purified monoclonal antibodies (named according to the hybridoma name). The activity and function of the purified anti-human BDCA-2 antibody were then tested.

### 2.1 Detection of the binding activity of hybridoma monoclonal antibody against human BDCA-2

Jurkat-hBDCA-2 cells expressing human BDCA-2 or Jurkat-cyno BDCA-2 cells expressing cynomolgus monkey BDCA-2 were placed in a 96-well U-bottom well plate at a density of 1×10⁶ cells/mL, 100 µL per well and the cells were centrifuged to form a pellet. First, the anti-BDCA-2 hybridoma antibody obtained in example 1 (17G6A2 and 164C11B8) and the positive control antibody BIIB059 (patent US9902775B2, which has the light chain shown in SEQ ID NO:3 and the heavy chain shown in SEQ ID NO: 4 disclosed in US9902775B2) were diluted to an initial working mass concentration of 10 µg/mL using FACS buffer. Then, the samples were serially diluted 1:3 using FACS buffer to obtain 10 dilutions. The negative control antibody IgG1 isotype (Biointron Co., Ltd., cat # B117901) was diluted to an initial working mass concentration of 10 µg/mL. Then, 100 µL of each dilution of antibody was added to the corresponding well of a 96-well plate, the cell pellet was resuspended and mixed, incubated at 4 °C for 1 hour, centrifuged to remove the supernatant, and the cells were washed three times with FACS buffer. A secondary antibody against mouse labeled with AlexaFlour-647 (Invitrogen, cat # A21445) was diluted 1:1000, 100 µL of the secondary antibody dilution was added to each well, the cell pellet was resuspended, incubated at 4 °C for 1 hour, centrifuged to remove the supernatant, the cells were washed three times, and resuspended with 100 µL of FACS buffer in each well. The mean fluorescence intensity (MFI) was analyzed by flow cytometry. The experimental data were analyzed using Graphpad Prism 8.0 software. The logarithm of the anti-BDCA-2 monoclonal antibody concentration was used as the x-axis and the corresponding MFI value was used as the y-axis. A four-parameter regression model was used to fit the dose-response curve of the anti-BDCA-2 monoclonal antibody and calculate the EC50. The obtained EC50 was the average value of multiple repeated experiments.

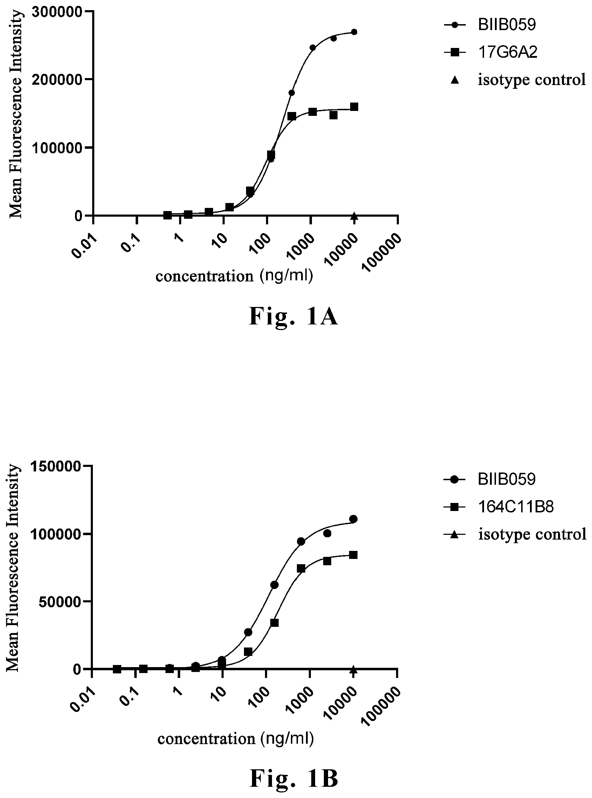

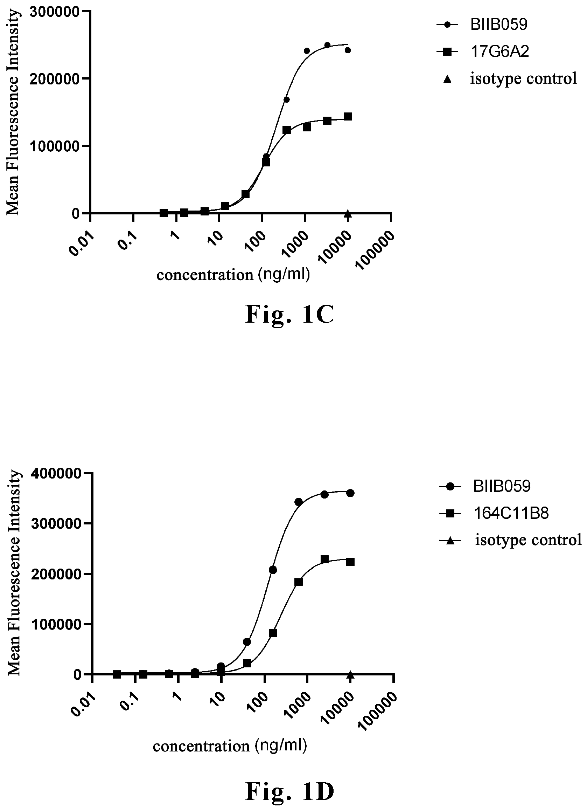

The results are shown in FIGS. 1A, 1B, 1C, 1D and Table 2. The EC₅₀ values for the binding activity of the 17G6A2 and 164C11B8 hybridoma supernatants with human BDCA-2 were 96.8 and 189.1 ng/mL, respectively, and the EC₅₀ values for the binding activity with cynomolgus BDCA-2 were 107.4 and 233.0 ng/mL, respectively. The EC₅₀ values for the binding activity of BIIB059 with human and cynomolgus monkey BDCA-2 were 224.2 and 207.3 ng/mL, respectively.

**Table 2. Binding activity of anti-BDCA-2 antibody with human BDCA-2 and cynomolgus monkey BDCA-2**

| Clone No. | Jurkat-hBDCA-2 | Jurkat-cynoBDCA-2 |
|---|---|---|
| | EC₅₀, ng/mL | EC₅₀, ng/mL |
| BIIB059 | 224.2 | 207.3 |
| 17G6A2 | 96.8 | 107.4 |
| 164C11B8 | 189.1 | 233.0 |

### 2.2. Anti-human BDCA-2 hybridoma monoclonal antibody inhibits IFNα production by PBMCs

Short fragments of single-stranded DNA, CpGA, stimulate pDC cells to produce type I interferons. BDCA-2 activation can inhibit the production of CpGA-induced type I interferon by pDCs. Anti-BDCA-2 antibody binds to BDCA-2, activates downstream BCR signaling, and inhibits pDCs from producing CpGA-induced type I interferon (PMID: 25762615, EMBO Mol Med. 2015 Apr;7(4): 464-76).In this section, CpGA was used to induce IFNα production in PBMCs and the inhibitory effect of anti-BDCA-2 hybridoma antibody on the produced type I interferon was examined.

The specific method is as follows: PBMCs were diluted to 2× 10⁷ cells/mL and were placed 50 µL per well in a 96-well U-plate. The anti-BDCA-2 hybridoma antibody and the positive control antibody BIIB059 were diluted to 20 µg/ml using RPMI-1640 complete medium, and then serially diluted 1:4 using RPMI-1640 complete medium to obtain 10 dilutions. The negative control antibody IgG1 isotype was diluted to 20 µg/ml. Then, 50 µL of each dilution of antibody was added to the corresponding well of a 96-well plate, and 1 µL of 100 µM CpGA (invivogen, cat #: tlrl-2216) was added to each well. The plate was then placed in a 5% CO₂ incubator at 37°C for 16 hours. 16 µL of supernatant was aspirated from each well and added to the corresponding well of the 384-well plate. The concentration of IFNα in the supernatant was detected according to the instructions using the Human IFNα kit (cisbio, cat #: 62HIFNAPEG). Specifically, 4 µL of IFNαd2 and 4 µL of crypate antibody mixture were added, incubated at room temperature for 2 hours, and relative light units (RLU) were read on cell culture plates using a microplate reader. The experimental data were analyzed using Graphpad Prism 8.0 software, with the logarithm of the anti-BDCA-2 monoclonal antibody concentration as the x-axis and the corresponding RLU value as the y-axis. A four-parameter equation regression model was selected to fit the dose-response curve of the anti-BDCA-2 monoclonal antibody.

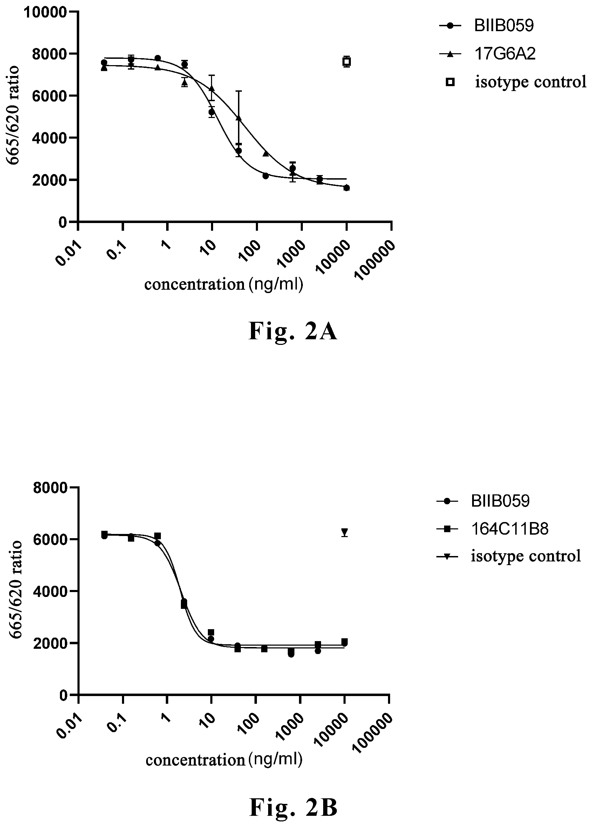

The results are shown in Figures 2A and 2B and Table 3. The EC₅₀ values of the purified 17G6A2 and 164C11B8 hybridoma antibodies for inhibiting CpGA-induced production of type I interferon by PBMCs were 54.2 and 2.0 ng/mL, respectively. The EC₅₀ values of BIIB059 for inhibiting CpGA-induced production of type I interferon by PBMCs were 13.2 and 2.0 ng/mL, respectively. The PBMCs used in Tables 3.1 and 3.2 were from different donors.

**Table 3.1. Inhibitory activity of anti-BDCA-2 antibody on CpGA-induced production of type I Interferon in human PBMC cells**

| Clone No. | EC₅₀, **ng/mL** |
|---|---|
| BIIB059 | 13.2 |
| 17G6A2 | 54.2 |

**Table 3.2. Inhibitory activity of anti-BDCA-2 antibody on CpGA-induced production of type I Interferon in human PBMC cells**

| Clone No. | EC₅₀, **ng/mL** |
|---|---|
| BIIB059 | 3.1 |
| 164C11B8 | 2.0 |

### Example 3: Construction, expression, and purification of chimeric antibody against BDCA-2.

Antibodies 17G6A2 and 164C11B8 generated from hybridoma clones 17G6A2 and 164C11B8 were sequenced to obtain the variable region sequences of the corresponding antibodies, which are shown in Table 4.1. Using conventional methods, the light and heavy chain variable regions (Table 4.1) of hybridoma antibodies 17G6A2 and 164C11B8 were constructed onto the human constant region (IgG1/K, Table 4.2) to obtain chimeric antibodies, which were named 17G6A2-hIgG1 and 164C11B8-hIgG1. Sequencing confirmed that sequences of the constructed chimeric hIgG1 antibodies were identical to those shown in Tables 4.1 and 4.2. The corresponding nucleic acids encoding the chimeric antibodies 17G6A2-hIgG1 and 164C11B8-hIgG1 were transfected into Expi293 cells for antibody expression, and then purified using a Protein A column. The specific method is as follows:
Antibody expression in Expi293 cells: One day before transfection, Expi293 cells were diluted to a density of 1.5 × 10⁶ cells/mL and cultured at 37°C, 8% CO₂, in a shaker at 120 rpm. On the second day, the cell density and viability were measured. The cell transfection density should be 3 × 10⁶ cells/mL and the cell viability should be greater than 95%. Preparation of PEI/plasmid complex: PEI (1 mg/mL, Polysciences, cat #: 24765-1) was gently mixed by inversion. The plasmid containing the encoded nucleic acid was diluted with OPM-293 CD05 Medium (Shanghai OPM Biosciences Co., Ltd., cat #: 81075-001), with a plasmid concentration of 1 µg/mL. The volume of the medium used to dilute the plasmid should be 1/20 of the transfection volume. Mixed gently, with a light to heavy chain ratio of 1:1.5. The PEI reagent was diluted with OPM-293 CD05 Medium. The volume of the medium used to dilute the PEI should be 1/20 of the transfection volume. Gently inverted to mix and incubated at room temperature for 5 minutes. The diluted PEI reagent was added to the diluted plasmid and gently inverted to mix. The PEI/plasmid complex was incubated at room temperature for 15 minutes, then the solution was slowly added dropwise into a transfer flask while gently rotating the flask during the addition. After transfection, the shake flask was placed in a shaker at 37°C and 8% CO₂ and incubated at 120 rpm. On the second day after transfection (24 hours after transfection), 10% OPM-293 ProFeed (Shanghai OPM Biosciences Co., Ltd., cat #: F081918) was added to the shake flask while gently rotating the flask during the addition. The shake flask was then returned to the shaker and the culture was continued for 5-7 days. The supernatant was subsequently harvested.

Purification of antibody by Protein A column: A gravity chromatography column was prepared, the top cover of the gravity chromatography column was opened, the gasket was placed at the bottom of the gravity column and pressed tightly. The packing material Protein A (Cytiva, cat #: 17549801) was prepared. The required packing material slurry volume was precisely calculated based on the target packing material volume and the slurry ratio, where the required packing material slurry volume = target packing material volume / packing material slurry ratio. The packing material was vortexed thoroughly to ensure that it was completely suspended. The packing material slurry was added to the bottom of the gravity chromatography column. At least 10 CV of equilibration buffer PBS was added to the gravity chromatography column. After equilibration, the outlet pH was measured. If the target pH was not reached, continued adding equilibration buffer until the target pH was reached. A certain volume of sample was slowly added into the gravity chromatography column. At least 10 CV of rinse buffer was added to the gravity chromatography column. 5CV of elution buffer (10-50 mM NaAc, pH 3.0-3.5) was slowly added to the gravity chromatography column, incubated for 3-5 minutes, and the eluent was collected. The elution step was repeated as needed. The pH of the eluent was adjusted to the target pH using neutralization buffer (1M Tris). Protein concentration was determined using Nanodrop. The buffer used to store the antibody was replaced with PBS by ultrafiltration.

**Table 4.1. CDR sequences of anti-BDCA-2 hybridoma antibody (determined using Kabat numbering)**

| Hybridoma antibodies | HCDR1 | HCDR2 | HCDR3 | LCDR1 | LCDR2 | LCDR3 |
|---|---|---|---|---|---|---|
| 17G6A2 | SYGMS (SEQ ID NO:1) | TISSGDSYTY YPDSVKG (SEQ ID NO:2) | QLYYSYGY YFDY (SEQ ID NO:3) | RASESVSFHG THLMH (SEQ ID NO:4) | AASNLES (SEQ ID NO:5) | QQSIEDPPT (SEQ ID NO:6) |
| 164C11B8 | TYTMS (SEQ ID NO:11) | TINSGGDNTY YPDSVKG (SEQ ID NO:12) | YRPYYYGS VLDY (SEQ ID NO:13) | KASQSVDYD GNSYVN (SEQ ID NO:14) | GASNLES (SEQ ID NO:15) | QQSDEDPW T (SEQ ID NO:16) |

### For hybridoma antibody 17G6A2,

the heavy chain variable region is: 
EVQLVESGGDLVKPGGSLKLSCAASGFTFSSYGMSWVRQTPDKRLEWVATISSGDSYTYYP
DSVKGRFTISRDNAKNTLYLQMSSLKSEDTAMYYCARQLYYSYGYYFDYWGQGTTLTVSS
(SEQ ID NO: 7)
the light chain variable region is:

DIVLTQSPASLAVSLGQRATISCRASESVSFHGTHLMHWYQQKPGQPPKLLIYAASNLESGV
PARFSGSGSETDFTLNIHPVEEEDAATYFCQQSIEDPPTFGGGTKLEIK (SEQ ID NO: 8)

### For hybridoma antibody 164C11B8,

the heavy chain variable region is: 
DVILVESGGGLVKPGGSLKLSCAASGITFSTYTMSWLRQTPEKRLEWVATINSGGDNTYYPD
SVKGRFTISRDNAKNNLYLQMSSLKSEDTALYYCATYRPYYYGSVLDYWGQGTTLTVSS
(SEQ ID NO: 17)
the light chain variable region is:

DIVLTQSPASLAVSLGQRATISCKASQSVDYDGNSYVNWYQQKPGQPPKLLIYGASNLESGI
PDRFSGSGSGTDFTLNIHPVEEEDGATYYCQQSDEDPWTFGGGTKLEIR (SEQ ID NO: 18)

**Table 4.2. Constant region sequences of chimeric and humanized antibodies**

| Sequence name | Heavy chain constant region | Light chain constant region |
|---|---|---|
| IgG1/K |  (SEQ ID NO:9) |  (SEQ ID NO:10) |

### Example 4. Functional characterization of anti-human BDCA-2 chimeric antibody

### 1. Binding Activity of anti-human BDCA-2 chimeric antibody

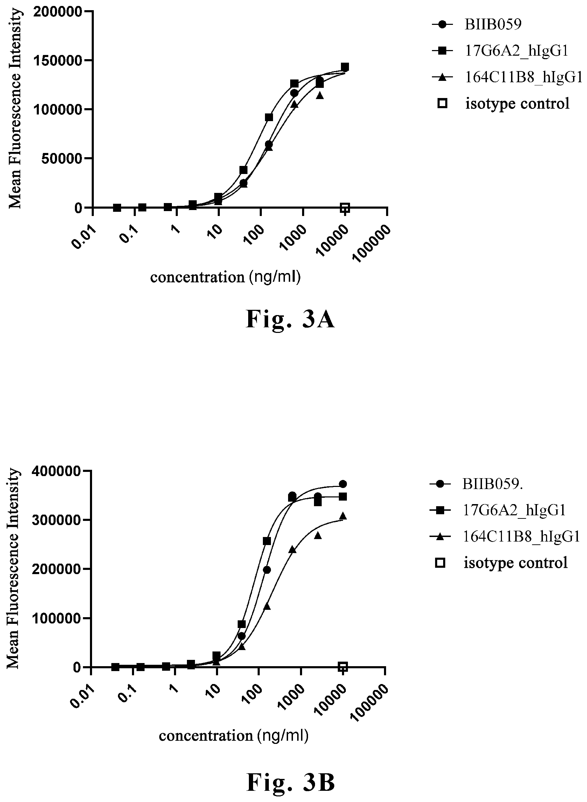

The binding activity of the anti- BDCA-2 chimeric antibody to human BDCA-2 (Figure 3A) and cynomolgus monkey BDCA-2 (Figure 3B) was detected using the method disclosed in Section 2.1 of Example 2, with BIIB059 as a control.

The results are shown in Figures 3A and 3B and Table 5. The EC₅₀ values for the binding activity of chimeric antibody 17G6A2_hIgG1 with human BDCA-2 and cynomolgus monkey BDCA-2 were 87.7 and 79.2 ng/mL, respectively. The EC₅₀ values for the binding activity of chimeric antibody 164C11B8 _hIgG1 with human BDCA-2 and cynomolgus monkey BDCA-2 were 215.4 and 210.0 ng/mL, respectively. The EC₅₀ values for the binding activity of BIIB059 with human and cynomolgus monkey BDCA-2 were 175.4 and 134.0 ng/mL, respectively.

**Table 5. Binding activity of anti-BDCA-2 antibody with human BDCA-2 and cynomolgus monkey BDCA-2**

| Number | Antibody | Jurkat-hBDCA-2 EC₅₀, ng/mL | Jurkat-cynoBDCA-2 EC₅₀, ng/mL |
|---|---|---|---|
| 1 | BIIB059 | 175.4 | 134.0 |
| 2 | 17G6A2_hIgG1 | 87.7 | 79.2 |
| 3 | 164C11B8_hIgG1 | 215.4 | 210.0 |

### 2. Anti-human BDCA-2 chimeric antibody inhibits CpGA-induced IFNα production in PBMCs

The activity of the anti-BDCA-2 chimeric antibody obtained in the present application in inhibiting IFNα production was detected using the method disclosed in Section 2.2 of Example 2, with BIIB059 as a control.

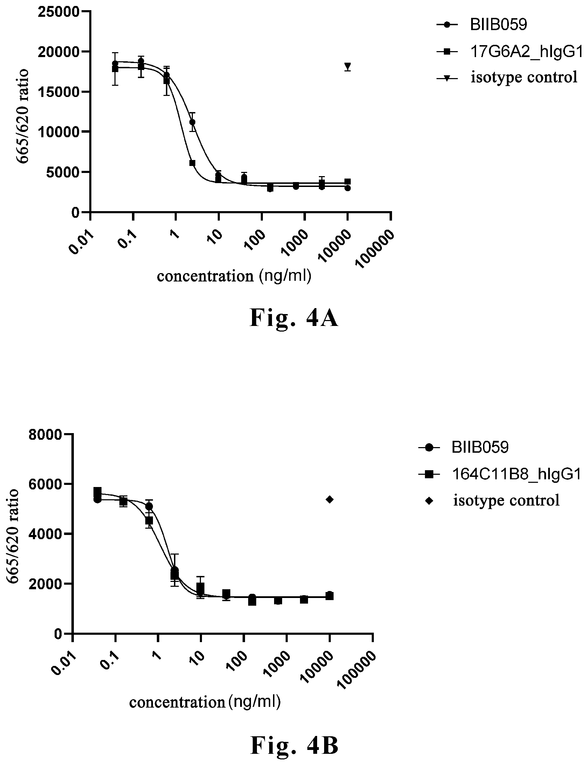

The results are shown in Figures 4A and 4B and Table 6. The EC₅₀ value for the inhibitory activity of chimeric antibody 17G6A2_hIgG1 was 1.3 ng/mL, the EC₅₀ value for the inhibitory activity of chimeric antibody 164C11B8_hIgG1 was 1.1 ng/mL, and the EC₅₀ value for the inhibitory activity of BIIB059 was 2.5 ng/mL.

**Table 6. Inhibition of CpGA-induced type 1 interferon in human PBMC cells by anti-BDCA-2 antibody**

| Clone No. | EC₅₀, **ng/mL** |
|---|---|
| BIIB059 | 2.5 |
| 17G6A2_hIgG1 | 1.3 |
| 164C11B8_hIgG1 | 1.1 |

### 3. Anti-human BDCA-2 chimeric antibody inhibits the release of IFNα from pDCs induced by immune complexes

Serum from patients with systemic lupus erythematosus (SLE) contains a large number of different anti-autoantibodies, primarily antinuclear antibodies. The antigen-antibody complex binds to the Fc receptor (CD32) on the surface of the pDC membrane, subsequently being internalized into the cell and activating TLR9, leading to the production of type I interferon. Anti-BDCA-2 antibody inhibits the production of type I interferon via this pathway (PMID: 25762615 EMBO Mol Med. 2015 Apr;7(4):464-76). In this section, we investigate whether the obtained chimeric antibody has the function of inhibiting the release of IFNα from pDC.

The specific method is as follows: pDC cells were sorted according to the instructions of the pDC sorting kit (STEM CELL, 17977). pDCs were diluted to 1× 10⁶ cells/mL and placed in 50 µL per well in a 96-well U-plate. The anti-BDCA-2 chimeric antibody and positive control antibody BIIB059 of the present application were diluted to 40 µg/ml using RPMI-1640 complete medium, and then serially diluted 1:4 using RMPI1640 complete medium to obtain 10 dilutions. The isotype control antibody was diluted to 40 µg/ml, and then 25 µL of each dilution of antibody was added to each well containing pDC cells. The antigen-antibody complex was prepared by mixing Sm/RNP Antigen (Raybiotech, MD-27-0014P) and human Anti-RNP Auto-antigen (Raybiotech, MD-14-0513) at a ratio of 1:2 and allowed to sit at room temperature for 30 minutes. Then, 25 µL of the immune complex was added to each well containing pDC cells and incubated in a 5% CO₂, 37°C incubator for 16 hours. 16 µL of supernatant was aspirated to the corresponding well in 384-well plate, and 4 µL of IFNαd2 and 4 µL of crypate antibody mixture (cisbo, 62HIFNAPEG) was added according to the kit instructions. Incubated at room temperature for 2 hours, and the relative light unit values were read on the cell culture plate using a microplate reader. The experimental data were analyzed using Graphpad Prism 8.0 software, with the logarithm of the anti-BDCA-2 monoclonal antibody concentration as the x-axis and the corresponding RLU value as the y-axis. A four-parameter equation regression model was used to fit the dose-response curve of the anti-BDCA-2 monoclonal antibody.

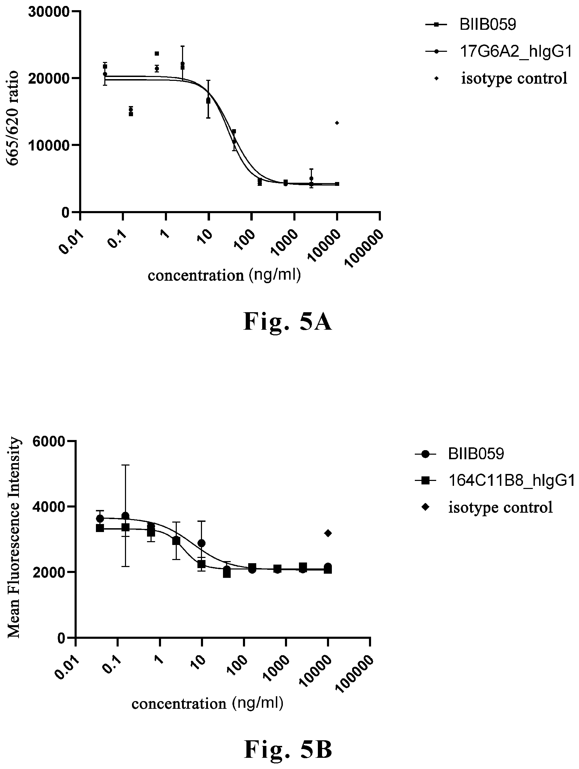

The results are shown in Figures 5A-B and Tables 7A-B. BIIB059, 17G6A2_hIgG1, and 164C11B8_hIgG1 all blocked the production of IFNα in pDCs induced by immune complexes. The EC₅₀ values for the inhibitory activity of the 17G6A2 chimeric antibody and BIIB059 were 29.2 ng/mL and 33.4 ng/mL, respectively; the EC₅₀ values for the inhibitory activity of the 164C11B8 chimeric antibody and BIIB059 were 3.6 ng/mL and 6.1 ng/mL, respectively. The pDCs used in Tables 7A and 7B were from different donors.

**Table 7A. Inhibitory activity of anti-BDCA-2 antibody on immune complex-induced type 1 interferon in human pDC cells**

| Number | Clone No. | EC₅₀, **ng/mL** |
|---|---|---|
| 1 | BIIB059 | 33.4 |
| 2 | 17G6A2_hIgG1 | 29.2 |

**Table 7B. Inhibitory activity of anti-BDCA-2 antibody on immune complex-inducd type 1 interferon in human pDC cells**

| Number | Clone No. | EC₅₀, **ng/mL** |
|---|---|---|
| 1 | BIIB059 | 6.1 |
| 2 | 164C11B8 _hIgG1 | 3.6 |

### 4. Chimeric antibody dose-dependently activates FcyR IIIa

Clinical studies have shown that anti-BDCA-2 antibodies can clear circulating pDCs through ADCC. The anti-BDCA-2 antibody-dependent activation of FcγRIIIa can be determined by incubating Jurkat-human FcγRIIIa(158V)-NFAT with cells expressing BDCA-2. The Fab terminus of the BDCA-2 antibody binds to the target site on target cells, while its Fc terminus binds to the FcγRIIIa receptor on effector cells, thereby activating the NFAT signaling pathway in effector cells. The ADCC activity of the antibody can be reflected by quantifying the luciferase produced during NFAT pathway activation. The specific method is as follows:
Jurkat-hBDCA-2 cells were centrifuged at 300 g for 5 minutes and the cell concentration was adjusted to 1.2 × 10⁶ cells/mL using RPMI-1640 medium. 50 µL of cells were seeded into each well of a white opaque plate to serve as target cells. The chimeric anti-BDCA-2 antibodies of the present application, positive control antibody, and negative control antibody IgG1 isotype were diluted to a concentration of 40 µg/ml using RPMI-1640 medium, and then serially diluted 4-fold with the medium. Jurkat-human FcγRIIIa (158V)-NFAT (Genomeditech (Shanghai) Co., Ltd., GM-C05619) cells were collected, centrifuged at 300 g for 5 minutes, the supernatant was discarded, and RPMI-1640 medium was added to adjust the cell concentration to 1.5×10⁶ cells/mL. 100 µL of the cells were added to each culture well containing the target cells, and simultaneously each well was added 50 µL of serially diluted anti-BDCA-2 antibody. Kept at 37°C and 5% CO₂ for overnight induction, and then at room temperature for at least 15 minutes for equilibration. 100 µL of luciferase substrate solution (Vazyme, DD1203) was added to each well, mixed, and reacted in the dark at room temperature for 5 minutes. The relative light unit values were read on the cell culture plate using a microplate reader. The experimental data were analyzed using Graphpad Prism 8.0 software, with the logarithm of the anti-BDCA-2 antibody concentration as the x-axis and the corresponding RLU value as the y-axis. A four-parameter regression model was selected to fit the dose-response curve of the anti-BDCA-2 antibody.

The results are shown in Figure 6 and Table 8. The 17G6A2 and 164C11B8 chimeric antibodies could activate Jurkat-human FcγRIIIa (158V)-NFAT reporter cells in a dose-dependent manner. The EC₅₀ value for the activation activity of the 17G6A2 chimeric antibody was 15.0 ng/mL, the EC₅₀ value for the activation activity of the 164C11B8 chimeric antibody was 11.9 ng/mL, and the EC₅₀ value for the activation activity of BIIB059 was 13.0 ng/mL.

**Table 8. EC₅₀ value of chimeric antibody to activate Jurkat-human FcγR IIIa (158V) -NFAT reporter cell line**

| Number | Chimeric antibody | EC₅₀, ng/mL |
|---|---|---|
| 1 | BIIB059 | 13.0 |
| 2 | 17G6A2 _hIgG1 | 15.0 |
| 3 | 164C11B8_hIgG1 | 11.9 |

### 5. Detection of chimeric antibody affinity

A sensor probe recognizing human IgG was placed into the Octet Red96e pre-wetted plate. The antibody was diluted to 5000 ng/mL with 0.02% PBST and 0.1% BSA, and 200 µL was added to each well of a black opaque plate. Human BDCA-2 protein was diluted to 400 nM (8800 ng/ml) with 0.02% PBST and 0.1% BSA, and then serially diluted 2-fold to obtain serially diluted human BDCA-2 protein. 200 µL of the diluted protein was then added to each well of a black opaque plate. The Octet Red96e-editing experimental method was run, and the binding signal values were read. The experimental data were analyzed using the Octet Data Analysis software to calculate various dynamic parameters.

The results are shown in Table 9. The affinity KD values of chimeric antibodies 17G6A2_hIgG1 and 164C11B8_hIgG1 were approximately 0.5 nM and 2.2 nM, respectively, and the affinity KD value of BIIB059 antibody was 1.9 nM.

**Table 9. Measurement of affinity of chimeric antibody**

| Chimeric antibody | KD (M) | kon(1/Ms) | kdis(1/s) |
|---|---|---|---|
| BIIB059 | 1.90E-09 | 7.99E+04 | 1.52E-04 |
| 17G6A2_hIgG1 | 5.19E-10 | 2.74E+05 | 1.42E-04 |
| 164C11B8_hIgG1 | 2.17E-09 | 1.56E+05 | 3.38E-04 |

### EXAMPLE 5. Construction, expression and purification of humanized anti-BDCA-2 antibody

The human germline gene with the highest homology to the murine sequence was selected as the acceptor framework, and the CDRs of the murine sequence (Table 4.1) were grafted into the human framework. According to the importance of amino acid, back mutation was carried out. Namely, some key amino acids in the human framework region were back mutated to the corresponding murine amino acids. Several variants of heavy and light chains were designed respectively, and complete antibody expression vectors were constructed. The heavy and light chains were combined for expression to obtain the humanized antibody protein.

### 1. Construction of humanized antibodies for anti-BDCA-2 antibody 17G6A2

The humanized heavy and light chain variable region sequences were designed as follows:

| Heavy and light chain variable region sequence numbering | Sequence |
|---|---|
| 17G6A2_ hzVH 01 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQLYYSYGYYFDYWGQGTTVTVSS (SEQ ID NO: 19) |
| 17G6A2_ hzVH 02 | EVQLVESGGGLVKPGGSLRLSCAASGFTFSSYGMSWVRQAPGKGLEW VATISSGDSYTYYPDSVKGRFTISRDNAKNSLYLQMNSLRAEDTAVYYC ARQLYYSYGYYFDYWGQGTTVTVSS (SEQ ID NO: 20) |
| 17G6A2_ hzVH 03 | EVQLVESGGGLVKPGGSLRLSCAASGFTFSSYGMSWVRQAPGKRLEW VATISSGDSYTYYPDSVKGRFTISRDNAKNSLYLQMNSLRAEDTAVYYC ARQLYYSYGYYFDYWGQGTTVTVSS (SEQ ID NO: 21) |
| 17G6A2_ hzVL 05 | DIVLTQSPASLAVSPGQRATITCRASESVSFHGTHLMHWYQQKPGQPPK LLIYAASNLESGVPARFSGSGSGTDFTLTINPVEAEDTANYYCQQSIEDP PTFGGGTKVEIK (SEQ ID NO: 22) |
| 17G6A2_ hzVL 06 | DIVLTQSPASLAVSPGQRATITCRASESVSFHGTHLMHWYQQKPGQPPK LLIYAASNLESGVPARFSGSGSGTDFTLTINPVEANDAANYYCQQSIEDP PTFGGGTKVEIK (SEQ ID NO: 23) |
| 17G6A2_ hzVL 07 | DIVLTQSPASLAVSPGQRATITCRASESVSFHGTHLMHWYQQKPGQPPK LLIYAASNLESGVPARFSGSGSETDFTLTINPVEAEDTANYYCQQSIEDPP TFGGGTKVEIK (SEQ ID NO: 24) |
| 17G6A2_ hzVL 08 | DIVLTQSPASLAVSPGQRATITCRASESVSFHGTHLMHWYQQKPGQPPK LLIYAASNLESGVPARFSGSGSETDFTLTINPVEANDAANYYCQQSIEDP PTFGGGTKVEIK (SEQ ID NO: 25) |

Humanized antibody numbering and its corresponding heavy and light chain variable region sequence numbers

| Antibody numbering | Heavy chain variable region | Light chain variable region |
|---|---|---|
| 17G6A2_hzH1L5_hIgG1 | 17G6A2_hzVH01 | 17G6A2_hzVL05 |
| 17G6A2_hzH1L6_hIgG1 | 17G6A2_hzVH01 | 17G6A2_hzVL06 |
| 17G6A2_hzH1L7_hIgG1 | 17G6A2_hzVH01 | 17G6A2_hzVL07 |
| 17G6A2_hzHL8_hIgG1 | 17G6A2_hzVH01 | 17G6A2_hzVL08 |
| 17G6A2_hzH2L5_hIgG1 | 17G6A2_hzVH02 | 17G6A2_hzVL05 |
| 17G6A2_hzH 2L6_hIgG1 | 17G6A2_hzVH02 | 17G6A2_hzVL06 |
| 17G6A2_hzH2L7_hIgG1 | 17G6A2_hzVH02 | 17G6A2_hzVL07 |
| 17G6A2_hzH2L8_hIgG1 | 17G6A2_hzVH02 | 17G6A2_hzVL08 |
| 17G6A2_hzH3L5_hIgG1 | 17G6A2_hzVH03 | 17G6A2_hzVL05 |
| 17G6A2_hzH3L6_hIgG1 | 17G6A2_hzVH03 | 17G6A2_hzVL06 |
| 17G6A2_hzH 3L7_hIgG1 | 17G6A2_hzVH03 | 17G6A2_hzVL07 |
| 17G6A2_hzH 3L8_hIgG1 | 17G6A2_hzVH03 | 17G6A2_hzVL08 |

### 2. Construction of humanized antibodies for anti-BDCA-2 antibody 164C11B8

The 164C11B8 humanized antibody variable region sequences are as follows:

| Heavy and light chain variable region sequence numbering | Sequence |
|---|---|
| 164C11B8_hzVH1 | EVQLLESGGGLVQPGGSLRLSCAASGFTFSTYTMSWVRQAP GKGLEWVSTINSGGDNTYYPDSVKGRFTISRDNSKNTLYLQ MNSLRAEDTAVYYCATYRPYYYGSVLDYWGQGTTVTVSS (SEQ ID NO: 26) |
| 164C11B8_hzVH2 | EVQLLESGGGLVQPGGSLRLSCAASGFTFSTYTMSWVRQAP GKGLEWVATINSGGDNTYYPDSVKGRFTISRDNSKNTLYLQ MNSLRAEDTAVYYCATYRPYYYGSVLDYWGQGTTVTVSS (SEQ ID NO: 27) |
| 164C11B8_hzVH3 | EVQLLESGGGLVQPGGSLRLSCAASGITFSTYTMSWVRQAP GKGLEWVATINSGGDNTYYPDSVKGRFTISRDNSKNTLYLQ MNSLRAEDTAVYYCATYRPYYYGSVLDYWGQGTTVTVSS (SEQ ID NO: 28) |
| 164C11B8_hzVH4 | EVQLLESGGGLVQPGGSLRLSCAASGITFSTYTMSWVRQAP GKRLEWVATINSGGDNTYYPDSVKGRFTISRDNSKNTLYLQ MNSLRAEDTAVYYCATYRPYYYGSVLDYWGQGTTVTVSS (SEQ ID NO: 29) |
| 164C11B8_hzVL1 | DIVMTQSPDSLAVSLGERATINCKASQSVDYDGNSYVNWY QQKPGQPPKLLIYGASNLESGVPDRFSGSGSGTDFTLTISSLQ AEDVAVYYCQQSDEDPWTFGGGTKVEIK (SEQ ID NO: 30) |
| 164C11B8_hzVL2 | DIVMTQSPDSLAVSLGERATINCKASQSVDYDGNSYVNWY QQKPGQPPKLLIYGASNLESGIPDRFSGSGSGTDFTLTISSLQ AEDVAVYYCQQSDEDPWTFGGGTKVEIK (SEQ ID NO: 31) |
| 164C11B8_hzVL3 | DIVLTQSPDSLAVSLGERATINCKASQSVDYDGNSYVNWY QQKPGQPPKLLIYGASNLESGIPDRFSGSGSGTDFTLTISSLQ AEDVAVYYCQQSDEDPWTFGGGTKVEIK (SEQ ID NO: 32) |

Humanized antibody numbering and its corresponding heavy and light chain variable region sequence numbers

| Name of antibody | Heavy chain variable region | Light chain variable region |
|---|---|---|
| 164C11B8_hzH1L1_hIgG1 | 164C11B8_hz V H1 | 164C11B8_hz V L1 |
| 164C11B8_hzH1L2 hIgG1 | 164C11B8_hz V H1 | 164C11B8_hz V L2 |
| 164C11B8_hzH1L3_hIgG1 | 164C11B8_hz V H1 | 164C11B8_hz V L3 |
| 164C11B8_hzH2L1_hIgG1 | 164C11B8_hz V H2 | 164C11B8_hz V L1 |
| 164C11B8_hzH2L2_hIgG1 | 164C11B8_hz V H2 | 164C11B8_hz V L2 |
| 164C11B8_hzH2L3_hIgG1 | 164C11B8_hz V H2 | 164C11B8_hz V L3 |
| 164C11B8_hzH3L1_hIgG1 | 164C11B8_hz V H3 | 164C11B8_hz V L1 |
| 164C11B8_hzH3L2_hIgG1 | 164C11B8_hz V H3 | 164C11B8_hz V L2 |
| 164C11B8_hzH3L3_hIgG1 | 164C11B8_hz V H3 | 164C11B8_hz V L3 |
| 164C11B8_hzH4L1_hIgG1 | 164C11B8_hz V H4 | 164C11B8_hz V L1 |
| 164C11B8_hzH4L2_hIgG1 | 164C11B8_hz V H4 | 164C11B8_hz V L2 |
| 164C11B8_hzH4L3_hIgG1 | 164C11B8_hz V H4 | 164C11B8_hz V L3 |

### 3. Expression and purification of the constructed humanized antibody

The light and heavy chain variable regions of the humanized antibody were connected with human constant regions (IgG 1/K, Table 4.2) by gene synthesis according to the disclosure of Example 3. The antibody was expressed in Expi293 cells and purified using Protein A. After purification, the buffer of the antibody is replaced with PBS through ultrafiltration buffer exchange.

### EXAMPLE 6. Binding activity of anti-BDCA-2 humanized antibody

### 1. Binding Activity of anti-BDCA-2 humanized antibody

The binding activity of the humanized anti-BDCA-2 antibodies obtained in Example 5 with human and cynomolgus monkey BDCA-2 was detected using the method disclosed in Section 2.1 of Example 2, with BIIB059 as a control.

The results are shown in Figures 7A, 7B, 7C, and 7D and Table 10. All humanized antibodies bound to human BDCA-2 and cynomolgus monkey BDCA-2, and the binding activity was comparable to that of BIIB059.

**Table 10. Binding Activity of anti-BDCA-2 antibodies with human BDCA-2 and cynomolgus monkey BDCA-2**

| Number | Humanized antibody | Jurkat-hBDCA-2 | Jurkat-cynoBDCA-2 |
|---|---|---|---|
| | | EC₅₀*, ng/mL | EC₅₀*, ng/mL |
| 1 | BIIB059 | 70.4 | 105.4 |
| 2 | 17G6A2_ hzH1L5_hIgG1 | 31.1 | 53.3 |
| 3 | 17G6A2_hzH1L6_hIgG1 | 55.3 | 99.7 |
| 4 | 17G6A2_ hzH1L7_hIgG1 | 59.5 | 94.4 |
| 5 | 17G6A2_ hzH1L8_hIgG1 | 44.2 | 78.1 |
| 6 | 17G6A2_ hzH2L5_hIgG1 | 179.8 | 94.3 |
| 7 | 17G6A2_hzH2L6_hIgG1 | 70.1 | 105.6 |
| 8 | 17G6A2_ hzH2L7_hIgG1 | 60.8 | 98.8 |
| 9 | 17G6A2_ hzH2L8_hIgG1 | 22.2 | 38.3 |
| 10 | 17G6A2_ hzH3L5_hIgG1 | 66.0 | 100.7 |
| 11 | 17G6A2_ hzH3L6_hIgG1 | 83.1 | 66.3 |
| 12 | 17G6A2_ hzH3L7_hIgG1 | 58.3 | 97.5 |
| 13 | 17G6A2_ hzH3L8_hIgG1 | 45.6 | 68.8 |
| 14 | 164C11B8_hzH1L1_hIgG1 | 42.9 | 157.8 |
| 15 | 164C11B8_hzH1L2_hIgG1 | 77.5 | 143.7 |
| 16 | 164C11B8_hzH1L3_hIgG1 | 113.0 | 193.4 |
| 17 | 164C11B8_hzH2L1_hIgG1 | 103.9 | 252.4 |
| 18 | 164C11B8_hzH2L2_hIgG | 105.6 | 255.9 |
| 19 | 164C11B8_hzH2L3_hIgG | 70.3 | 114.8 |
| 20 | 164C11B8_hzH3L1_hIgG | 67.5 | N/A |
| 21 | 164C11B8_hzH3L2_hIgG | 112.3 | 154.1 |
| 22 | 164C11B8_hzH3L3_hIgG | 73.0 | 83.7 |
| 23 | 164C11B8_hzH4L1_hIgG | 191.4 | 309.7 |
| 24 | 164C11B8_hzH4L2_hIgG | 61.1 | 103.7 |
| 25 | 164C11B8_hzH4L3_hIgG | 109.7 | 160.3 |

| | | | |
|---|---|---|---|
| *Average value of multiple experiments; NT: Not detected. N/A: Not applicable; no valid EC₅₀ was obtained. | | | |

### 2. Humanized antibodies do not bind to Jurkat cells

Next, following the method described in Section 2.1 of Example 2, the ability of the humanized antibody obtained in the present application to bind to blank Jurkat cells was tested.

As shown in Figure 7E, neither the humanized antibody 17G6A2 nor BIIB059 showed binding to Jurkat cells, and their binding patterns were similar to that of the negative control isotype.

### 3. Detection of affinity of humanized antibody

The affinity of the anti-BDCA-2 humanized antibody was detected using the method disclosed in Section 5 of Example 4. The results are shown in Table 11. The KD values of the 17G6A2 humanized antibodies were comparable to that of BIIB059. The KD values of 17G6A2_hzH1L5_hIgG1, 17G6A2_hzH1L6_hIgG1, and 17G6A2_hzH1L7_hIgG1 were 4.4E-09, 5.9E-09, and 2.9E-09 M, respectively; and the KD value of BIIB059 was 3.9E-09 M.

**Table 11. Measurement of the affinity of humanized antibodies to human BDCA-2**

| Number | Clone No. | KD (M) | kon(1/Ms) | kdis(1/s) |
|---|---|---|---|---|
| 1 | BIIB059 | 3.9E-09 | 7.53E+04 | 2.91E-04 |
| 2 | 17G6A2_hIgG1 | 1.4E-09 | 1.94E+05 | 2.63E-04 |
| 3 | 17G6A2_hzH1L5_hIgG1 | 4.4E-09 | 1.11E+05 | 4.90E-04 |
| 4 | 17G6A2 _hzH1L6_hIgG1 | 5.9E-09 | 1.20E+05 | 7.05E-04 |
| 5 | 17G6A2_hzH1L7_hIgG1 | 2.9E-09 | 1.79E+05 | 5.23E-04 |
| 6 | 17G6A2_hzH1L8_hIgG1 | 6.3E-09 | 1.38E+05 | 8.72E-04 |
| 7 | 17G6A2_hzH2L5_hIgG1 | 3.2E-09 | 1.76E+05 | 5.67E-04 |
| 8 | 17G6A2_hzH2L6_hIgG1 | 3E-09 | 1.01E+05 | 3.09E-04 |
| 9 | 17G6A2_hzH2L7_hIgG1 | 1.8E-09 | 1.74E+05 | 3.11E-04 |
| 10 | 17G6A2_hzH2L8_hIgG1 | 1.7E-09 | 1.54E+05 | 2.61E-04 |

### EXAMPLE 7. Functional activity of anti-BDCA-2 humanized antibody

### 1. Anti-BDCA-2 humanized antibody inhibits IFNα production by PBMCs

The activity of the anti-BDCA-2 humanized antibody in inhibiting IFNα production by human PBMCs was detected using the method in Section 2.2 of Example 2, with BIIB059 as a control.

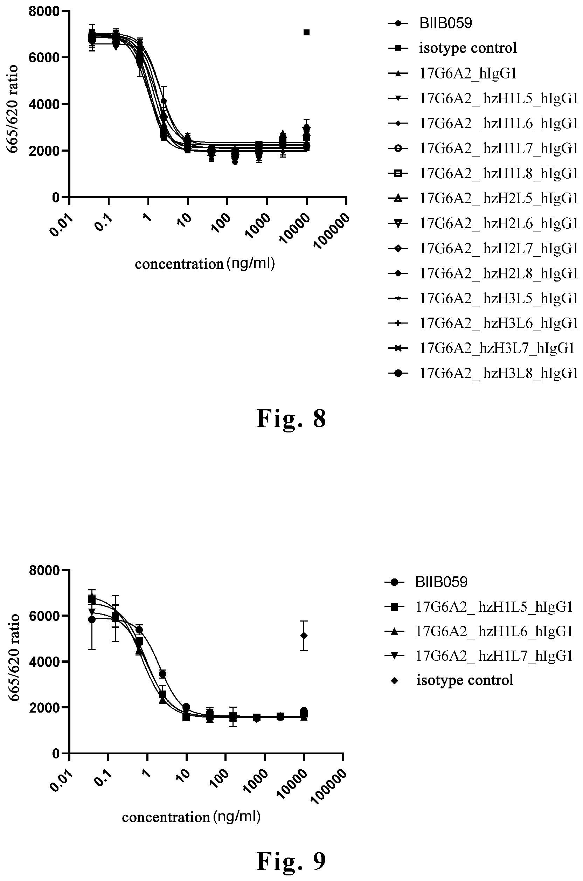

The results are shown in Figure 8 and Table 12. Both BIIB059 and humanized antibodies of 17G6A2 blocked CpGA-induced IFNα production.

**Table 12. Inhibitory activity of anti-BDCA-2 antibody in CpGA-induced type I interferon production in human PBMC cells.**

| Number | Clone No. | EC₅₀, ng/mL |
|---|---|---|
| 1 | BIIB059 | 2.1 |
| 2 | 17G6A2_hzH1L5 _hIgG1 | 1.0 |
| 3 | 17G6A2_hzH1L6_hIgG1 | 1.4 |
| 4 | 17G6A2_hzH1L7_hIgG1 | 1.1 |
| 5 | 17G6A2_hzH1L8_hIgG1 | 1.1 |
| 6 | 17G6A2_hzH2L5_hIgG1 | 1.4 |
| 7 | 17G6A2_hzH2L6_hIgG1 | 1.7 |
| 8 | 17G6A2_hzH2L7_hIgG1 | 1.1 |
| 9 | 17G6A2_hzH2L8_hIgG1 | 1.1 |
| 10 | 17G6A2_hzH3L5_hIgG1 | 1.4 |
| 11 | 17G6A2_hzH3L6_hIgG1 | 1.7 |
| 12 | 17G6A2_hzH3L7_hIgG1 | 1.1 |
| 13 | 17G6A2_hzH3L8_hIgG1 | 1.1 |

### 2. Anti-BDCA-2 humanized antibody inhibits IFNα production by pDC cells

The activity of the anti-BDCA-2 humanized antibodies in inhibiting IFNα production by human pDC cells was detected using the method described in Section 3 of Example 4, with BIIB059 as a control.

The results are shown in Figure 9 and Table 13. Both BIIB059 and humanized antibodies of 17G6A2 could block the IFNα production induced by immune complexes. The EC₅₀ values of the 17G6A2 humanized antibodies 17G6A2_hzH1L5_hIgG1, 17G6A2_hzH1L6_hIgG1, and 17G6A2_hzH1L7_hIgG1 were 0.9 ng/mL, 0.7 ng/mL and 1.0 ng/mL, respectively, and the EC₅₀ value of BIIB059 was 2.1 ng/mL.

**Table 13. Inhibitory activity of anti-BDCA-2 antibody on immune complex-induced type I interferon production by human pDC cells**

| Number | Clone No. | EC₅₀, ng/mL |
|---|---|---|
| 1 | BIIB059 | 2.1 |
| 2 | 17G6A2_hzH1L5_hIgG1 | 0.9 |
| 3 | 17G6A2_hzH1L6_hIgG1 | 0.7 |
| 4 | 17G6A2_hzH1L7_hIgG1 | 1.0 |

### 3. Binding of anti-BDCA-2 humanized antibody to pDCs

pDC cells were sorted according to the instructions of the pDC sorting kit (STEM CELL, 17977). pDCs were diluted to 1× 10⁵ cells/mL and placed in 50 µL per well in a 96-well U-plate. The anti-BDCA-2 humanized antibody, positive control antibody BIIB059, and isotype hIgG1 were diluted to 20 µg/ml using RPMI-1640 complete medium, and then serially diluted 1:5 using RPMI-1640 complete medium to obtain 8 dilutions. Each was added in 50 µL/well into a well containing pDC cells. Cells were incubated at 4°C for 1 hour, centrifuged to form a pellet, and washed three times with FACS buffer. The anti-human secondary antibody (Invitrogen, cat #: A21445) labeled with AlexaFlour-647 was diluted 1:1000. The cell pellet was resuspended with 100 µL/well of the secondary antibody dilution buffer, mixed by pipetting, and incubated at 4°C for 1 hour. The cells were then centrifuged to form a pellet, washed three times, and resuspended in 100 µL/well of FACS buffer. Median Fluorescence Intensity (MFI) was analyzed by a flow cytometry. The experimental data were analyzed using Graphpad Prism 8.0 software, with the logarithm of the anti-BDCA-2 monoclonal antibody concentration as the x-axis and the corresponding MFI value as the y-axis. A four-parameter equation regression model was used to fit the dose-response curve of the anti-BDCA-2 monoclonal antibody to calculate EC₅₀.

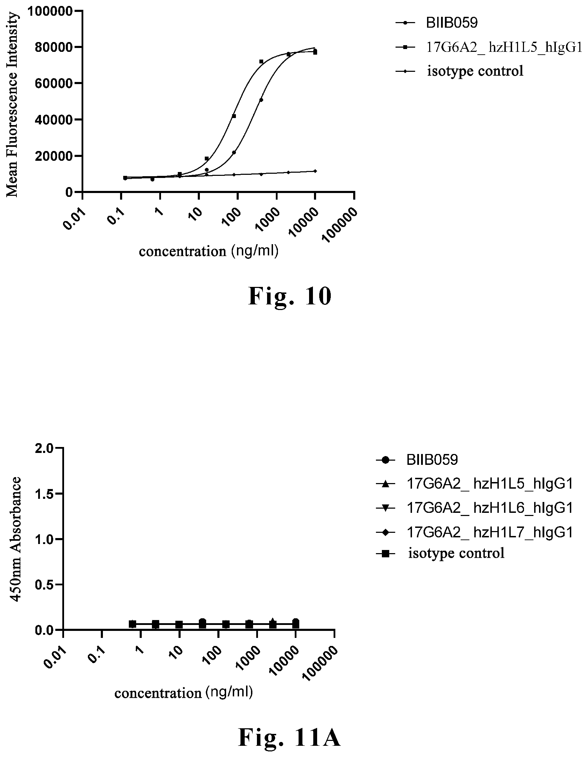

The results are shown in Figure 10 and Table 14. The EC₅₀ value of the binding activity of the 17G6A2 humanized antibody to human pDCs was 77.6 ng/mL, and the EC₅₀ value of the binding activity of the BIIB059 to human pDCs was 280.6 ng/mL.

**Table 14. Binding Activity of anti-BDCA-2 antibody to human pDCs**

| Number | Humanized antibody | EC₅₀, ng/mL |
|---|---|---|
| 1 | BIIB059 | 280.6 |
| 2 | 17G6A2_hzH1L5_hIgG1 | 77.6 |

### 4. Anti-BDCA-2 antibody does not bind to CLEC4A, CLEC4E, CLEC4D or CLEC6A

BDCA-2, CLEC4A, CLEC4E, CLEC4D and CLEC6A all belong to the C-type lectin receptor (PMID: 29553870, MAbs. 2018 May/Jun; 10(4):651-663). Therefore, the inventors continued to investigate whether the obtained humanized antibodies have cross-reactivity with other receptors. The binding activity of BDCA-2 antibodies with human CLEC4A, CLEC4E, CLEC4D, and CLEC6A was detected using the following ELISA method. The above-mentioned proteins were diluted to 1 µg/ml with ELISA coating buffer (solarbio, C1055), respectively, 100 µL was added to the plate (corning: 9018) and incubated overnight at 4°C. The next day, the plate was washed three times with PBST, blocked with 1% bovine serum albumin (BSA) protein (Sangon Biotech, 9048-46-8) at 37°C for 2 hours, and washed three times with PBST. The anti-BDCA-2 antibodies were serially 4-fold diluted, starting with 10 µg/ml. 100 µL/well of each dilution was added to the wells, incubated at 37 °C for 1 hour, and washed three times with PBST. A secondary antibody (abcam, ab98535) was added for 30 minutes, and the plate was washed three times with PBST. Substrate A (BD OptEIA, 51-2606KC) and substrate B (BD OptEIA, 51-2607KC) were mixed at a ratio of 1:1, and 100 µL was added to each well. The mixture was reacted for 20 minutes, and then stop solution (solarbio, C1058) was added.

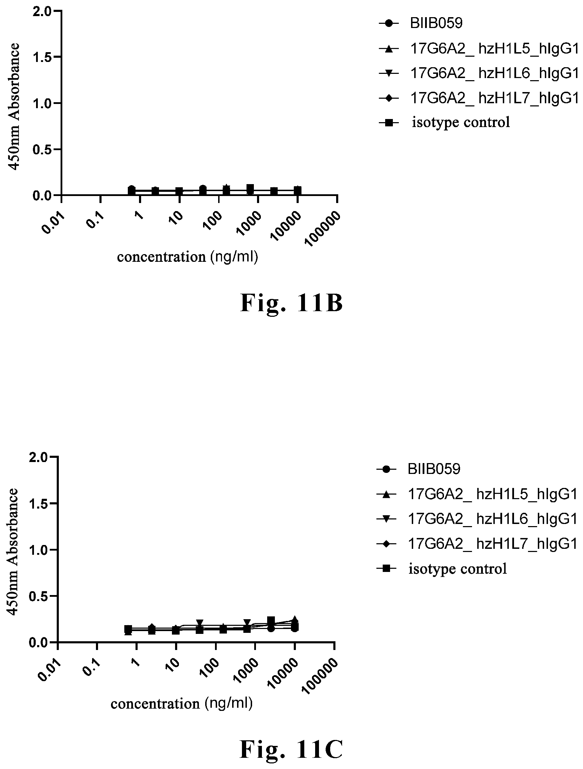

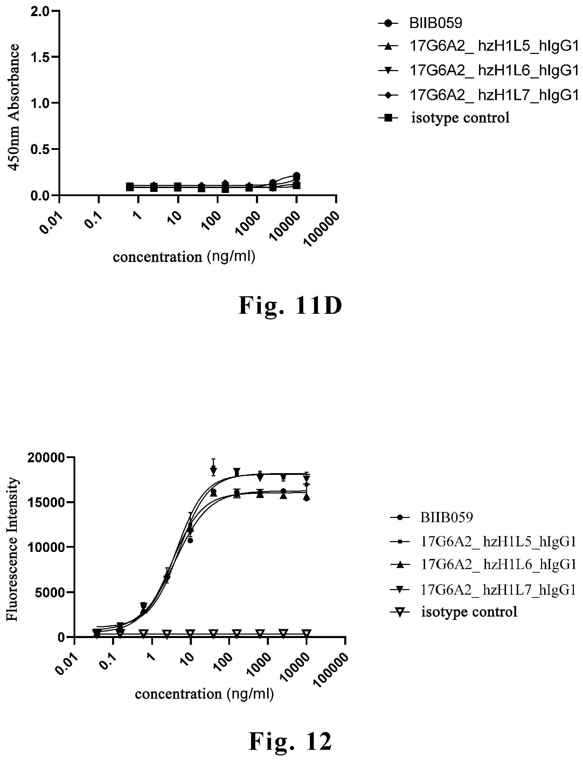

As shown in Figures 11A-D, the 17G6A2 humanized antibodies prepared in the present application did not bind to protein CLEC4A, CLEC4E, CLEC4D or CLEC6A.

### 5. Affinity of humanized antibody to cyno BDCA-2

The test was performed according to the method shown in Section 5 of Example 4. The antibody was diluted to 5000 ng/mL with 0.02% PBST and 0.1% BSA. The cynomolgus monkey BDCA-2 protein (acro, CLC-C52H4) was diluted to 400 nM with 0.02% PBST and 0.1% BSA, and then serially diluted by 2-fold to obtain the gradient dilutions of cynomolgus monkey BDCA-2 protein. 200 µL was added to each well in a black opaque plate. The Octet Red96e-editing experimental method was run, and the binding signal values were read. The experimental data were analyzed using the Octet Data Analysis software to calculate various dynamic parameters.

The results are shown in Table 15. The affinity KD values of humanized antibodies 17G6A2_hzH1L5_hIgG1, 17G6A2_hzH1L6_hIgG1 and 17G6A2_hzH1L7_hIgG1 to cynomolgus monkey BDCA-2 protein were 5.7, 5.6 nM, and 4.8 nM, respectively, and the affinity KD value of BIIB059 antibody was 1.1 nM.

**Table 15. Determination of the affinity of humanized antibody to cyno BDCA-2**

| Number | Humanized antibody | KD (M) | kon(1/Ms) | kdis(1/s) |
|---|---|---|---|---|
| 1 | BIIB059 | 1.1E-09 | 1.0E+05 | 1.1E-04 |
| 2 | 17G6A2_hzH1L5_hIgG1 | 5.7E-09 | 1.2E+05 | 6.7E-04 |
| 3 | 17G6A2_hzH1L6_hIgG1 | 5.6E-09 | 1.1E+05 | 6.5E-04 |
| 4 | 17G6A2_hzH1L7_hIgG1 | 4.8E-09 | 1.3E+05 | 6.4E-04 |

### 6. 17G6A2 humanized antibodies dose-dependently activate FcγR IIIa

Following the method described in Section 4 of Example 4, dose-dependent FcγRIIIa activation of the 17G6A2 humanized antibodies was determined using the Jurkat-human FcγRIIIa (158V)-NFAT reporter assay, and the results are shown in Figure 12 and Table 16. The EC₅₀ values for the activation activity of antibodies 17G6A2_hzH1L5_hIgG1, 17G6A2_hzH1L6_hIgG1, and 17G6A2_hzH1L7_hIgG1 were 4.2, 3.2, and 5.0 ng/ml, respectively, and the EC₅₀ value for the activation activity of BIIB059 was 4.0 ng/ml.

**Table 16. EC₅₀ values for activating Jurkat-human FcγR IIIa (158V)-NFAT cell line of humanized antibody**

| Number | Humanized antibody | EC₅₀, **ng/mL** |
|---|---|---|
| 1 | BIIB059 | 4.0 |
| 2 | 17G6A2_hzH1L5_hIgG1 | 4.2 |
| 3 | 17G6A2_hzH1L6_hIgG1 | 3.2 |
| 4 | 17G6A2_hzH1L7_hIgG1 | 5.0 |

### EXAMPLE 8. Efficacy of antibodies of the present disclosure for treating SLE animals

Establish an imiquimod cream (Nanbo, Tianfang Pharmaceutical Co., Ltd., batch number: 220703001)-induced SLE skin injury model in immunodeficient pDC mice using immunodeficient NOD scid mice (NOD.CB17 - Prkdcscid/NcrCrl, Vital River Laboratory Animal Technology Co., Ltd., catalog number: 406). The specific method is as follows: Imiquimod cream was applied to a 1.5cm × 1.5cm marked area on the back of the mouse every 12 hours, for a total of three administrations. Antibodies were administered via tail vein once during the second administration of Imiquimod cream, and human pDCs (3 x 10⁵ cells/100 µL) were administered via tail vein 12 hours after the last administration of Imiquimod cream. Samples were taken and observed at the endpoint to detect pathological changes in mouse skin and to measure the hematological parameters. The results of immunohistochemical staining and flow cytometry data indicated that both 17G6A2_hzH1L5_hIgG1 and the positive control drug BIIB059 inhibited the trend of epidermal thickening in mice, reduced the numbers of pDC cells and CD123+ cells in mouse skin, and suppressed the number of MX1 positive cells downstream of the IFNα. The trends of the two are comparable.

### EXAMPLE 9. Antibodies of the present disclosure induce the internalization of BDCA2 on pDCs in cynomolgus monkeys

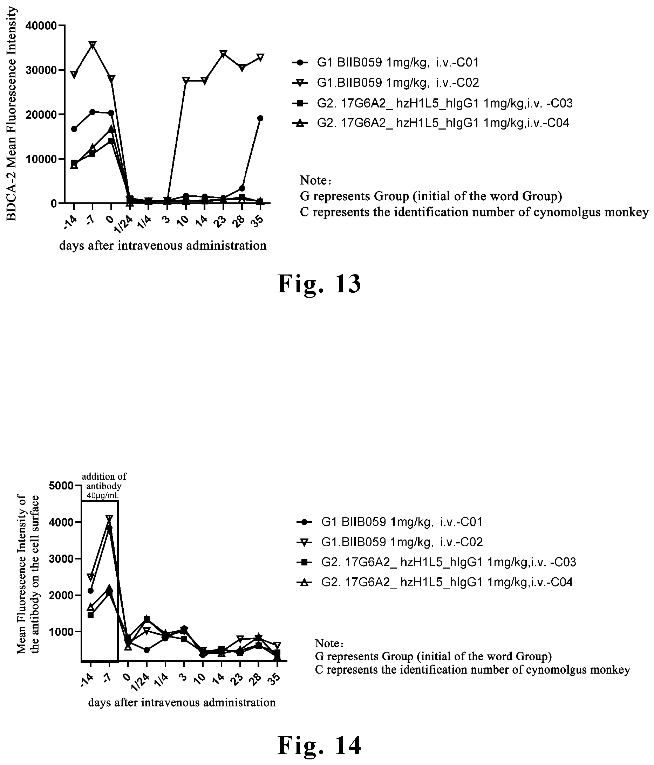

2 mL of EDTA-anticoagulated whole blood samples were collected from four cynomolgus monkeys (male, weighing about 4 kg, 2 years old, Suzhou Xihua New Drug Development Co., Ltd.) 2 weeks and 1 week before drug administration to detect the proportion of pDCs in the whole blood. Based on the proportion of pDCs and body weight, cynomolgus monkeys were randomly divided into two groups, and were intravenously injected with 1 mg/kg of 17G6A2_hzH1L5_hIgG1 (experimental group) or BIIB059 (control group). Anticoagulated whole blood samples were collected from each group of cynomolgus monkeys at 0 hours before drug administration and at 1 hour, 6 hours, 3 days, 10 days, 14 days, 21 days, 28 days and 35 days after drug administration. The content of BDCA-2 antigen and the content of the binding of 17G6A2_hzH1L5_hIgG1 or BIIB059 on the surface of pDC cells were detected in each blood sample to reflect the antibody-induced BDCA-2 protein internalization. The specific method is as follows:
1) Direct method for detecting BDCA-2 expression: Anticoagulated bloods from cynomolgus monkeys at 14 days, 7 days, and 0 hours before drug administration, and at 1 hour, 6 hours, 3 days, 10 days, 14 days, 21 days, 28 days, and 35 days after drug administration were treated with erythrocyte lysis (BD Biosciences, cat #: 555899). Cells in peripheral blood samples from cynomolgus monkeys were then detected using anti-CD14-APC/Cy7 (Biolegend, cat #: 301820), anti-CD20-PE (Biolegend, cat #: 302306), anti-HLA-DR-BV510 (Biolegend, cat #: 307646), and anti-CD123-FITC (BD Biosciences, cat #: 558663). CD14⁻CD20⁻HLA-DR⁺CD123⁺ cells were identified as pDC cells. BDCA2 on the pDC surface in the samples obtained at various time points was detected using AF647-labeled 17G6A2_hzH1L5_hIgG1 or BIIB059. Cells were collected using CytoFlex and the data were analyzed on CytExpert. The results are shown in Figure 13.
2) Indirect labeling method for detecting 17G6A2_hzH11L5_hIgG1 or BIIB059 bound to the pDC surface.

Anticoagulated blood samples at 14 days and 7 days before drug administration were treated with erythrocyte lysis and then 40 µg/mL of 17G6A2_hzH1L5_hIgG1 or BIIB059 was added, respectively, to detect their maximum binding capacity on the pDC surface. The pDCs were stained and detected according to the method described in part 1), and 17G6A2_hzH1L5_hIgG1 or BIIB059 bound to the pDC surface was indirectly detected using AF647-labeled anti-human IgG secondary antibody.

Anticoagulated blood samples from cynomolgus monkeys at 0 hours before administration and at 1 hour, 6 hours, 3 days, 10 days, 14 days, 21 days, 28 days, and 35 days after administration were treated with erythrocyte lysis and stained according to the method described in part 1) to detect pDCs. The 17G6A2_hzH1L5_hIgG1 or BIIB059 bound to the pDC surface was indirectly detected using AF647-labeled anti-human IgG secondary antibody. Cells were collected using CytoFlex and analyzed on CytExpert, and the results are shown in Figure 14.

As shown in Figure 13 (direct labeling method), BDCA-2 expression was almost undetectable in pDC cells of cynomolgus monkeys from 1 hour to 3 days after administration of 17G6A2_hzH1L5_hIgG1 or BIIB059, indicating that BDCA-2 on the surface of pDC cells was occupied by 17G6A2_hzH1L5_hIgG1 or BIIB059, or internalization of BDCA-2 was induced. In the BIIB059 treatment group, the expression of BDCA-2 was detectable again on the surface of pDC cells at 10 days and 28 days after administration, respectively. However, in the 17G6A2_hzH1L5_hIgG1 treatment group, the expression of BDCA-2 was still undetectable until 35 days after administration. From 10 days to 28 days after administration, BDCA-2 was no longer occupied by the drug and was restored to the cell surface. Therefore, AF647 - labeled BIIB059 could bind to the unoccupied BDCA-2, and thus the expression of BDCA-2 was detected. In the 17G6A2_hzH1L5_hIgG1 treatment group, it is possible that the drug still occupied BDCA-2 and remained inside the cells, so no signal was detected.

As shown in Figure 14 (indirect labeling method), when 17G6A2_hzH1L5_hIgG1 or BIIB059 was added to anticoagulated blood collected 2 weeks and 1 week before administration, the corresponding antibodies 17G6A2_hzH1L5_hIgG1 or BIIB059 binding to BDCA-2 could be detected on pDC cells, indicating that pDC cells expressed high level of BDCA-2. However, after administration, 17G6A2_hzH1L5_hIgG1 or BIIB059 was almost undetectable on pDC cells.

In summary, both 17G6A2_hzH1L5_hIgG1 and BIIB059 induced internalization of BDCA-2 on the surface of pDCs in cynomolgus monkeys, with 17G6A2_hzH1L5_hIgG1 showing a more sustained effect on BDCA-2 internalization or inhibition than BIIB059.

### Description of the sequence listing

### Chimeric antibody 17G6A2

Heavy chain: 
EVQLVESGGDLVKPGGSLKLSCAASGFTFSSYGMSWVRQTPDKRLEWVATISSGDSYTYYPDSVKGRFTI
SRDNAKNTLYLQMSSLKSEDTAMYYCARQLYYSYGYYFDYWGQGTTLTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 33)
Light chain:

DIVLTQSPASLAVSLGQRATISCRASESVSFHGTHLMHWYQQKPGQPPKLLIYAASNLESGV
PARFSGSGSETDFTLNIHPVEEEDAATYFCQQSIEDPPTFGGGTKLEIKRTVAAPSVFIFPPSDE
QLKSGTASVVCLLNNFYPREAKVQWKVDNALQSGNSQESVTEQDSKDSTYSLSSTLTLSKA
DYEKHKVYACEVTHQGLSSPVTKSFNRGEC (SEQ ID NO: 34)

### Chimeric antibody 164C11B8

Heavy chain: 
DVILVESGGGLVKPGGSLKLSCAASGITFSTYTMSWLRQTPEKRLEWVATINSGGDNTYYPD
SVKGRFTISRDNAKNNLYLQMSSLKSEDTALYYCATYRPYYYGSVLDYWGQGTTLTVSS
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFS
CSVMHEALHNHYTQKSLSLSPGK (SEQ ID NO: 35)
Light chain:

DIVLTQSPASLAVSLGQRATISCKASQSVDYDGNSYVNWYQQKPGQPPKLLIYGASNLESGI
PDRFSGSGSGTDFTLNIHPVEEEDGATYYCQQSDEDPWTFGGGTKLEIRRTVAAPSVFIFPPS
DEQLKSGTASVVCLLNNFYPREAKVQWKVDNALQSGNSQESVTEQDSKDSTYSLSSTLTLS
KADYEKHKVYACEVTHQGLSSPVTKSFNRGEC (SEQ ID NO: 36)

### Corresponding heavy and light chain sequences of humanized antibodies

| Humanized antibodies | Sequence |
|---|---|
| 17G6A2_ hzH1L5_hIg G1 | Heavy chain: |
| | EVQLVESGGGLVKPGGSLRLSCAASGFTFSSYGMSWVRQAPGKGLEWV STISSGDSYTYYPDSVKGRFTISRDNAKNSLYLQMNSLRAEDTAVYYCAR |
| | QLYYSYGYYFDYWGQGTTVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLF PPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK (SEQ ID NO: 37) |
| | Light chain: |
| | DIVLTQSPASLAVSPGQRATITCRASESVSFHGTHLMHWYQQKPGQPPKL LIYAASNLESGVPARFSGSGSGTDFTLTINPVEAEDTANYYCQQSIEDPPT FGGGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVT KSFNRGEC (SEQ ID NO: 38) |
| | Heavy chain: |
| | EVQLVESGGGLVKPGGSLRLSCAASGFTFSSYGMSWVRQAPGKGLEWV STISSGDSYTYYPDSVKGRFTISRDNAKNSLYLQMNSLRAEDTAVYYCAR QLYYSYGYYFDYWGQGTTVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLF PPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK (SEQ ID NO: 37) |
| | Light chain: |
| 17G6A2_ hzH1L6_hIg G1 | DIVLTQSPASLAVSPGQRATITCRASESVSFHGTHLMHWYQQKPGQPPKLLIYAASN LESGVPARFSGSGSGTDFTLTINPVEANDAANYYCQQSIEDPPTFGGGTKVEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 39) |
| | Heavy chain: |
| 17G6A2hzH1L7_hIg G1 | EVQLVESGGGLVKPGGSLRLSCAASGFTFSSYGMSWVRQAPGKGLEWV STISSGDSYTYYPDSVKGRFTISRDNAKNSLYLQMNSLRAEDTAVYYCAR QLYYSYGYYFDYWGQGTTVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLF PPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK (SEQ ID NO: 37) |
| | Light chain: |
| | DIVLTQSPASLAVSPGQRATITCRASESVSFHGTHLMHWYQQKPGQPPKLLIYAASN LESGVPARFSGSGSETDFTLTINPVEAEDTANYYCQQSIEDPPTFGGGTKVEIKRTVAA PSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC (SEQ ID NO: 40) |
| | Heavy chain: |
| | EVQLVESGGGLVKPGGSLRLSCAASGFTFSSYGMSWVRQAPGKGLEWV STISSGDSYTYYPDSVKGRFTISRDNAKNSLYLQMNSLRAEDTAVYYCAR QLYYSYGYYFDYWGQGTTVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLF PPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK (SEQ ID NO: 37) |
| | Light chain: |
| 17G6A2_ hzH1L8_hIg G1 | DIVLTQSPASLAVSPGQRATITCRASESVSFHGTHLMHWYQQKPGQPPKLLIYAASN LESGVPARFSGSGSETDFTLTINPVEANDAANYYCQQSIEDPPTFGGGTKVEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 41) |
| | Heavy chain: |
| | EVQLVESGGGLVKPGGSLRLSCAASGFTFSSYGMSWVRQAPGKGLEWV ATISSGDSYTYYPDSVKGRFTISRDNAKNSLYLQMNSLRAEDTAVYYCA RQLYYSYGYYFDYWGQGTTVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK (SEQ ID NO: 42) |
| | Light chain: |
| 17G6A2_ hzH2L5_hIg G1 | DIVLTQSPASLAVSPGQRATITCRASESVSFHGTHLMHWYQQKPGQPPKLLIYAASN LESGVPARFSGSGSGTDFTLTINPVEAEDTANYYCQQSIEDPPTFGGGTKVEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 38) |
| | Heavy chain: |
| | EVQLVESGGGLVKPGGSLRLSCAASGFTFSSYGMSWVRQAPGKGLEWV ATISSGDSYTYYPDSVKGRFTISRDNAKNSLYLQMNSLRAEDTAVYYCA RQLYYSYGYYFDYWGQGTTVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK (SEQ ID NO: 42) |
| | Light chain: |
| 17G6A2_ hzH2L6_hIg G1 | DIVLTQSPASLAVSPGQRATITCRASESVSFHGTHLMHWYQQKPGQPPKLLIYAASN LESGVPARFSGSGSGTDFTLTINPVEANDAANYYCQQSIEDPPTFGGGTKVEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 39) |
| | Heavy chain: |
| | EVQLVESGGGLVKPGGSLRLSCAASGFTFSSYGMSWVRQAPGKGLEWV ATISSGDSYTYYPDSVKGRFTISRDNAKNSLYLQMNSLRAEDTAVYYCA RQLYYSYGYYFDYWGQGTTVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK (SEQ ID NO: 42) |
| | Light chain: |
| 17G6A2_ hzH2L7_hIg G1 | DIVLTQSPASLAVSPGQRATITCRASESVSFHGTHLMHWYQQKPGQPPKLLIYAASN LESGVPARFSGSGSETDFTLTINPVEAEDTANYYCQQSIEDPPTFGGGTKVEIKRTVAA PSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC (SEQ ID NO: 40) |
| | Heavy chain: |
| | EVQLVESGGGLVKPGGSLRLSCAASGFTFSSYGMSWVRQAPGKGLEWV ATISSGDSYTYYPDSVKGRFTISRDNAKNSLYLQMNSLRAEDTAVYYCA RQLYYSYGYYFDYWGQGTTVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK (SEQ ID NO: 42) |
| | Light chain: |
| 17G6A2_ hzH2L8_hIg G1 | DIVLTQSPASLAVSPGQRATITCRASESVSFHGTHLMHWYQQKPGQPPKLLIYAASN LESGVPARFSGSGSETDFTLTINPVEANDAANYYCQQSIEDPPTFGGGTKVEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 41) |
| | Heavy chain: |
| | EVQLVESGGGLVKPGGSLRLSCAASGFTFSSYGMSWVRQAPGKRLEWV ATISSGDSYTYYPDSVKGRFTISRDNAKNSLYLQMNSLRAEDTAVYYCA RQLYYSYGYYFDYWGQGTTVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK (SEQ ID NO: 43) |
| | Light chain: |
| 17G6A2_ hzH3L5_hIg G1 | DIVLTQSPASLAVSPGQRATITCRASESVSFHGTHLMHWYQQKPGQPPKLLIYAASN LESGVPARFSGSGSGTDFTLTINPVEAEDTANYYCQQSIEDPPTFGGGTKVEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 38) |
| | Heavy chain: |
| 17G6A2_ hzH3L6_hIg G1 | EVQLVESGGGLVKPGGSLRLSCAASGFTFSSYGMSWVRQAPGKRLEWV ATISSGDSYTYYPDSVKGRFTISRDNAKNSLYLQMNSLRAEDTAVYYCA RQLYYSYGYYFDYWGQGTTVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK (SEQ ID NO: 43) |
| | Light chain: |
| | DIVLTQSPASLAVSPGQRATITCRASESVSFHGTHLMHWYQQKPGQPPKL LIYAASNLESGVPARFSGSGSGTDFTLTINPVEANDAANYYCQQSIEDPPT FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEV THQGLSSPVTKSFNRGEC (SEQ ID NO: 39) |
| | Heavy chain: |
| | EVQLVESGGGLVKPGGSLRLSCAASGFTFSSYGMSWVRQAPGKRLEWV ATISSGDSYTYYPDSVKGRFTISRDNAKNSLYLQMNSLRAEDTAVYYCA RQLYYSYGYYFDYWGQGTTVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK (SEQ ID NO: 43) |
| | Light chain: |
| 17G6A2_ hzH3L7_hIg G1 | DIVLTQSPASLAVSPGQRATITCRASESVSFHGTHLMHWYQQKPGQPPKL LIYAASNLESGVPARFSGSGSETDFTLTINPVEAEDTANYYCQQSIEDPPTF GGGTKVEIKRTVAAPSVFIFPPSDEQLKSGTASVVCLLNNFYPREAKVQW KVDNALQSGNSQESVTEQDSKDSTYSLSSTLTLSKADYEKHKVYACEVT HQGLSSPVTKSFNRGEC (SEQ ID NO: 40) |
| | Heavy chain: |
| 17G6A2_ hzH3L8_hIg G1 | EVQLVESGGGLVKPGGSLRLSCAASGFTFSSYGMSWVRQAPGKRLEWV ATISSGDSYTYYPDSVKGRFTISRDNAKNSLYLQMNSLRAEDTAVYYCA RQLYYSYGYYFDYWGQGTTVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK (SEQ ID NO: 43) |
| | Light chain: |
| | DIVLTQSPASLAVSPGQRATITCRASESVSFHGTHLMHWYQQKPGQPPKL LIYAASNLESGVPARFSGSGSETDFTLTINPVEANDAANYYCQQSIEDPPT FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEV THQGLSSPVTKSFNRGEC (SEQ ID NO: 41) |

## Claims

1. An antibody or antigen-binding fragment thereof that binds to BDCA-2, comprising:
1) the three heavy chain CDRs (HCDR1, HCDR2, HCDR3) comprised in the heavy chain variable region as set forth in SEQ ID NO: 7 and/or the three light chain CDRs (LCDR1, LCDR2, LCDR3) comprised in the light chain variable region as set forth in SEQ ID NO: 8; or
2) the three heavy chain CDRs (HCDR1, HCDR2, HCDR3) comprised in the heavy chain variable region as set forth in SEQ ID NO: 17 and/or the three light chain CDRs (LCDR1, LCDR2, LCDR3) comprised in the light chain variable region as set forth in SEQ ID NO: 18.

2. The antibody or antigen-binding fragment thereof that binds to BDCA-2 of claim 1, comprising heavy chain variable region CDRs (HCDR 1, HCDR2, HCDR3) and/or light chain variable region CDRs (LCDR1, LCDR2, LCDR3), wherein
1) HCDR1 comprises the sequence as set forth in SEQ ID NO: 1, or a sequence comprising one or more amino acid substitutions (e.g., conservative substitutions), deletions or insertions, or any combination thereof, relative to SEQ ID NO: 1, or consists of SEQ ID NO: 1; HCDR2 comprises the sequence as set forth in SEQ ID NO: 2, or a sequence comprising one or more amino acid substitutions (e.g., conservative substitutions), deletions or insertions, or any combination thereof, relative to SEQ ID NO: 2, or consists of SEQ ID NO: 2; HCDR3 comprises the sequence as set forth in SEQ ID NO: 3, or a sequence comprising one or more amino acid substitutions (*e.g*., conservative substitutions), deletions or insertions, or any combination thereof, relative to SEQ ID NO: 3, or consists of SEQ ID NO: 3; LCDR1 comprises the sequence as set forth in SEQ ID NO: 4, or a sequence comprising one or more amino acid substitutions (*e.g*., conservative substitutions), deletions or insertions, or any combination thereof, relative to SEQ ID NO: 4, or consists of SEQ ID NO: 4; LCDR2 comprises the sequence as set forth in SEQ ID NO: 5, or a sequence comprising one or more amino acid substitutions (*e.g*., conservative substitutions), deletions or insertions, or any combination thereof, relative to SEQ ID NO: 5, or consists of SEQ ID NO: 5; LCDR3 comprises the sequence as set forth in SEQ ID NO: 6, or a sequence comprising one or more amino acid substitutions (*e.g*., conservative substitutions), deletions or insertions, or any combination thereof, relative to SEQ ID NO: 6, or consists of SEQ ID NO: 6; or
2) HCDR1 comprises the sequence as set forth in SEQ ID NO: 11, or a sequence comprising one or more amino acid substitutions (*e.g*., conservative substitutions), deletions or insertions, or any combination thereof, relative to SEQ ID NO: 11, or consists of SEQ ID NO: 11; HCDR2 comprises the sequence as set forth in SEQ ID NO: 12, or a sequence comprising one or more amino acid substitutions (*e.g*., conservative substitutions), deletions or insertions, or any combination thereof, relative to SEQ ID NO: 12, or consists of SEQ ID NO: 12; HCDR3 comprises the sequence as set forth in SEQ ID NO: 13, or a sequence comprising one or more amino acid substitutions (*e.g*., conservative substitutions), deletions or insertions, or any combination thereof, relative to SEQ ID NO: 13, or consists of SEQ ID NO: 13; LCDR1 comprises the sequence as set forth in SEQ ID NO: 14, or a sequence comprising one or more amino acid substitutions (*e.g*., conservative substitutions), deletions or insertions, or any combination thereof, relative to SEQ ID NO: 14, or consists of SEQ ID NO: 14; LCDR2 comprises the sequence as set forth in SEQ ID NO: 15, or a sequence comprising one or more amino acid substitutions (*e.g*., conservative substitutions), deletions or insertions, or any combination thereof, relative to SEQ ID NO: 15, or consists of SEQ ID NO: 15; LCDR3 comprises the sequence as set forth in SEQ ID NO: 16, or a sequence comprising one or more amino acid substitutions (*e.g*., conservative substitutions), deletions or insertions, or any combination thereof, relative to SEQ ID NO: 16, or consists of SEQ ID NO: 16.

3. The antibody or antigen-binding fragment thereof that binds to BDCA-2 of claim 1 or 2, comprising a heavy chain variable region and/or a light chain variable region, wherein:
1) the heavy chain variable region comprises or consists of the amino acid sequence as set forth in SEQ ID NO: 7 or an amino acid sequence having at least 90% identity with the amino acid sequence of SEQ ID NO: 7; and the light chain variable region comprises or consists of the amino acid sequence as set forth in SEQ ID NO: 8 or an amino acid sequence having at least 90% identity with the amino acid sequence of SEQ ID NO: 8;
2) the heavy chain variable region comprises or consists of the amino acid sequence as set forth in SEQ ID NO: 17 or an amino acid sequence having at least 90% identity with the amino acid sequence of SEQ ID NO: 17; and the light chain variable region comprises or consists of the amino acid sequence as set forth in SEQ ID NO: 18 or an amino acid sequence having at least 90% identity with the amino acid sequence of SEQ ID NO: 18;
3) the heavy chain variable region comprises or consists of the amino acid sequence as set forth in SEQ ID NO: 19 or an amino acid sequence having at least 90% identity with the amino acid sequence of SEQ ID NO: 19; and the light chain variable region comprises or consists of the amino acid sequence as set forth in SEQ ID NO: 22 or an amino acid sequence having at least 90% identity with the amino acid sequence of SEQ ID NO: 22;
4) the heavy chain variable region comprises or consists of the amino acid sequence as set forth in SEQ ID NO: 19 or an amino acid sequence having at least 90% identity with the amino acid sequence of SEQ ID NO: 19; and the light chain variable region comprises or consists of the amino acid sequence as set forth in SEQ ID NO: 23 or an amino acid sequence having at least 90% identity with the amino acid sequence of SEQ ID NO: 23;
5) the heavy chain variable region comprises or consists of the amino acid sequence as set forth in SEQ ID NO: 19 or an amino acid sequence having at least 90% identity with the amino acid sequence of SEQ ID NO: 19; and the light chain variable region comprises or consists of the amino acid sequence as set forth in SEQ ID NO: 24 or an amino acid sequence having at least 90% identity with the amino acid sequence of SEQ ID NO: 24;
6) the heavy chain variable region comprises or consists of the amino acid sequence as set forth in SEQ ID NO: 19 or an amino acid sequence having at least 90% identity with the amino acid sequence of SEQ ID NO: 19; and the light chain variable region comprises or consists of the amino acid sequence as set forth in SEQ ID NO: 25 or an amino acid sequence having at least 90% identity with the amino acid sequence of SEQ ID NO: 25;
7) the heavy chain variable region comprises or consists of the amino acid sequence as set forth in SEQ ID NO: 20 or an amino acid sequence having at least 90% identity with the amino acid sequence of SEQ ID NO: 20; and the light chain variable region comprises or consists of the amino acid sequence as set forth in SEQ ID NO: 22 or an amino acid sequence having at least 90% identity with the amino acid sequence of SEQ ID NO: 22;
8) the heavy chain variable region comprises or consists of the amino acid sequence as set forth in SEQ ID NO: 20 or an amino acid sequence having at least 90% identity with the amino acid sequence of SEQ ID NO: 20; and the light chain variable region comprises or consists of the amino acid sequence as set forth in SEQ ID NO: 23 or an amino acid sequence having at least 90% identity with the amino acid sequence of SEQ ID NO: 23;
9) the heavy chain variable region comprises or consists of the amino acid sequence as set forth in SEQ ID NO: 20 or an amino acid sequence having at least 90% identity with the amino acid sequence of SEQ ID NO: 20; and the light chain variable region comprises or consists of the amino acid sequence as set forth in SEQ ID NO: 24 or an amino acid sequence having at least 90% identity with the amino acid sequence of SEQ ID NO: 24;
10) the heavy chain variable region comprises or consists of the amino acid sequence as set forth in SEQ ID NO: 20 or an amino acid sequence having at least 90% identity with the amino acid sequence of SEQ ID NO: 20; and the light chain variable region comprises or consists of the amino acid sequence as set forth in SEQ ID NO: 25 or an amino acid sequence having at least 90% identity with the amino acid sequence of SEQ ID NO: 25;
11) the heavy chain variable region comprises or consists of the amino acid sequence as set forth in SEQ ID NO: 21 or an amino acid sequence having at least 90% identity with the amino acid sequence of SEQ ID NO: 21; and the light chain variable region comprises or consists of the amino acid sequence as set forth in SEQ ID NO: 22 or an amino acid sequence having at least 90% identity with the amino acid sequence of SEQ ID NO: 22;
12) the heavy chain variable region comprises or consists of the amino acid sequence as set forth in SEQ ID NO: 21 or an amino acid sequence having at least 90% identity with the amino acid sequence of SEQ ID NO: 21; and the light chain variable region comprises or consists of the amino acid sequence as set forth in SEQ ID NO: 23 or an amino acid sequence having at least 90% identity with the amino acid sequence of SEQ ID NO: 23;
13) the heavy chain variable region comprises or consists of the amino acid sequence as set forth in SEQ ID NO: 21 or an amino acid sequence having at least 90% identity with the amino acid sequence of SEQ ID NO: 21; and the light chain variable region comprises or consists of the amino acid sequence as set forth in SEQ ID NO: 24 or an amino acid sequence having at least 90% identity with the amino acid sequence of SEQ ID NO: 24;
14) the heavy chain variable region comprises or consists of the amino acid sequence as set forth in SEQ ID NO: 21 or an amino acid sequence having at least 90% identity with the amino acid sequence of SEQ ID NO: 21; and the light chain variable region comprises or consists of the amino acid sequence as set forth in SEQ ID NO: 25 or an amino acid sequence having at least 90% identity with the amino acid sequence of SEQ ID NO: 25;
15) the heavy chain variable region comprises or consists of the amino acid sequence as set forth in SEQ ID NO: 26 or an amino acid sequence having at least 90% identity with the amino acid sequence of SEQ ID NO: 26; and the light chain variable region comprises or consists of the amino acid sequence as set forth in SEQ ID NO: 30 or an amino acid sequence having at least 90% identity with the amino acid sequence of SEQ ID NO: 30;
16) the heavy chain variable region comprises or consists of the amino acid sequence as set forth in SEQ ID NO: 26 or an amino acid sequence having at least 90% identity with the amino acid sequence of SEQ ID NO: 26; and the light chain variable region comprises or consists of the amino acid sequence as set forth in SEQ ID NO: 31 or an amino acid sequence having at least 90% identity with the amino acid sequence of SEQ ID NO: 31;
17) the heavy chain variable region comprises or consists of the amino acid sequence as set forth in SEQ ID NO: 26 or an amino acid sequence having at least 90% identity with the amino acid sequence of SEQ ID NO: 26; and the light chain variable region comprises or consists of the amino acid sequence as set forth in SEQ ID NO: 32 or an amino acid sequence having at least 90% identity with the amino acid sequence of SEQ ID NO: 32;
18) the heavy chain variable region comprises or consists of the amino acid sequence as set forth in SEQ ID NO: 27 or an amino acid sequence having at least 90% identity with the amino acid sequence of SEQ ID NO: 27; and the light chain variable region comprises or consists of the amino acid sequence as set forth in SEQ ID NO: 30 or an amino acid sequence having at least 90% identity with the amino acid sequence of SEQ ID NO: 30;
19) the heavy chain variable region comprises or consists of the amino acid sequence as set forth in SEQ ID NO: 27 or an amino acid sequence having at least 90% identity with the amino acid sequence of SEQ ID NO: 27; and the light chain variable region comprises or consists of the amino acid sequence as set forth in SEQ ID NO: 31 or an amino acid sequence having at least 90% identity with the amino acid sequence of SEQ ID NO: 31;
20) the heavy chain variable region comprises or consists of the amino acid sequence as set forth in SEQ ID NO: 27 or an amino acid sequence having at least 90% identity with the amino acid sequence of SEQ ID NO: 27; and the light chain variable region comprises or consists of the amino acid sequence as set forth in SEQ ID NO: 32 or an amino acid sequence having at least 90% identity with the amino acid sequence of SEQ ID NO: 32;
21) the heavy chain variable region comprises or consists of the amino acid sequence as set forth in SEQ ID NO: 28 or an amino acid sequence having at least 90% identity with the amino acid sequence of SEQ ID NO: 28; and the light chain variable region comprises or consists of the amino acid sequence as set forth in SEQ ID NO: 30 or an amino acid sequence having at least 90% identity with the amino acid sequence of SEQ ID NO: 30;
22) the heavy chain variable region comprises or consists of the amino acid sequence as set forth in SEQ ID NO: 28 or an amino acid sequence having at least 90% identity with the amino acid sequence of SEQ ID NO: 28; and the light chain variable region comprises or consists of the amino acid sequence as set forth in SEQ ID NO: 31 or an amino acid sequence having at least 90% identity with the amino acid sequence of SEQ ID NO: 31;
23) the heavy chain variable region comprises or consists of the amino acid sequence as set forth in SEQ ID NO: 28 or an amino acid sequence having at least 90% identity with the amino acid sequence of SEQ ID NO: 28; and the light chain variable region comprises or consists of the amino acid sequence as set forth in SEQ ID NO: 32 or an amino acid sequence having at least 90% identity with the amino acid sequence of SEQ ID NO: 32;
24) the heavy chain variable region comprises or consists of the amino acid sequence as set forth in SEQ ID NO: 29 or an amino acid sequence having at least 90% identity with the amino acid sequence of SEQ ID NO: 29; and the light chain variable region comprises or consists of the amino acid sequence as set forth in SEQ ID NO: 30 or an amino acid sequence having at least 90% identity with the amino acid sequence of SEQ ID NO: 30;
25) the heavy chain variable region comprises or consists of the amino acid sequence as set forth in SEQ ID NO: 29 or an amino acid sequence having at least 90% identity with the amino acid sequence of SEQ ID NO: 29; and the light chain variable region comprises or consists of the amino acid sequence as set forth in SEQ ID NO: 31 or an amino acid sequence having at least 90% identity with the amino acid sequence of SEQ ID NO: 31; or
26) the heavy chain variable region comprises or consists of the amino acid sequence as set forth in SEQ ID NO: 29 or an amino acid sequence having at least 90% identity with the amino acid sequence of SEQ ID NO: 29; and the light chain variable region comprises or consists of the amino acid sequence as set forth in SEQ ID NO: 32 or an amino acid sequence having at least 90% identity with the amino acid sequence of SEQ ID NO: 32.

4. The antibody or antigen-binding fragment thereof that binds to BDCA-2 of any one of claims 1-3, comprising a heavy chain and/or a light chain, wherein:
1) the heavy chain comprises the amino acid sequence as set forth in SEQ ID NO: 33 or an amino acid sequence having at least 90% identity with the amino acid sequence of SEQ ID NO: 33, or consists of SEQ ID NO: 33; and the light chain comprises the amino acid sequence as set forth in SEQ ID NO: 34 or an amino acid sequence having at least 90% identity with the amino acid sequence of SEQ ID NO: 34, or consists of SEQ ID NO: 34;
2) the heavy chain comprises the amino acid sequence as set forth in SEQ ID NO: 35 or an amino acid sequence having at least 90% identity with the amino acid sequence of SEQ ID NO: 35, or consists of SEQ ID NO: 35; and the light chain comprises the amino acid sequence as set forth in SEQ ID NO: 36 or an amino acid sequence having at least 90% identity with the amino acid sequence of SEQ ID NO: 36, or consists of SEQ ID NO: 36;
3) the heavy chain comprises the amino acid sequence as set forth in SEQ ID NO: 37 or an amino acid sequence having at least 90% identity with the amino acid sequence of SEQ ID NO: 37, or consists of SEQ ID NO: 37; and the light chain comprises the amino acid sequence as set forth in SEQ ID NO: 38 or an amino acid sequence having at least 90% identity with the amino acid sequence of SEQ ID NO: 38, or consists of SEQ ID NO: 38;
4) the heavy chain comprises the amino acid sequence as set forth in SEQ ID NO: 37 or an amino acid sequence having at least 90% identity with the amino acid sequence of SEQ ID NO: 37, or consists of SEQ ID NO: 37; and the light chain comprises the amino acid sequence as set forth in SEQ ID NO: 39 or an amino acid sequence having at least 90% identity with the amino acid sequence of SEQ ID NO: 39, or consists of SEQ ID NO: 39;
5) the heavy chain comprises the amino acid sequence as set forth in SEQ ID NO: 37 or an amino acid sequence having at least 90% identity with the amino acid sequence of SEQ ID NO: 37, or consists of SEQ ID NO: 37; and the light chain comprises the amino acid sequence as set forth in SEQ ID NO: 40 or an amino acid sequence having at least 90% identity with the amino acid sequence of SEQ ID NO: 40, or consists of SEQ ID NO: 40;
6) the heavy chain comprises the amino acid sequence as set forth in SEQ ID NO: 37 or an amino acid sequence having at least 90% identity with the amino acid sequence of SEQ ID NO: 37, or consists of SEQ ID NO: 37; and the light chain comprises the amino acid sequence as set forth in SEQ ID NO: 41 or an amino acid sequence having at least 90% identity with the amino acid sequence of SEQ ID NO: 41, or consists of SEQ ID NO: 41;
7) the heavy chain comprises the amino acid sequence as set forth in SEQ ID NO: 42 or an amino acid sequence having at least 90% identity with the amino acid sequence of SEQ ID NO: 42, or consists of SEQ ID NO: 42; and the light chain comprises the amino acid sequence as set forth in SEQ ID NO: 38 or an amino acid sequence having at least 90% identity with the amino acid sequence of SEQ ID NO: 38, or consists of SEQ ID NO: 38;
8) the heavy chain comprises the amino acid sequence as set forth in SEQ ID NO: 42 or an amino acid sequence having at least 90% identity with the amino acid sequence of SEQ ID NO: 42, or consists of SEQ ID NO: 42; and the light chain comprises the amino acid sequence as set forth in SEQ ID NO: 39 or an amino acid sequence having at least 90% identity with the amino acid sequence of SEQ ID NO: 39, or consists of SEQ ID NO: 39;
9) the heavy chain comprises the amino acid sequence as set forth in SEQ ID NO: 42 or an amino acid sequence having at least 90% identity with the amino acid sequence of SEQ ID NO: 42, or consists of SEQ ID NO: 42; and the light chain comprises the amino acid sequence as set forth in SEQ ID NO: 40 or an amino acid sequence having at least 90% identity with the amino acid sequence of SEQ ID NO: 40, or consists of SEQ ID NO: 40;
10) the heavy chain comprises the amino acid sequence as set forth in SEQ ID NO: 42 or an amino acid sequence having at least 90% identity with the amino acid sequence of SEQ ID NO: 42, or consists of SEQ ID NO: 42; and the light chain comprises the amino acid sequence as set forth in SEQ ID NO: 41 or an amino acid sequence having at least 90% identity with the amino acid sequence of SEQ ID NO: 41, or consists of SEQ ID NO: 41;
11) the heavy chain comprises the amino acid sequence as set forth in SEQ ID NO: 43 or an amino acid sequence having at least 90% identity with the amino acid sequence of SEQ ID NO: 43, or consists of SEQ ID NO: 43; and the light chain comprises the amino acid sequence as set forth in SEQ ID NO: 38 or an amino acid sequence having at least 90% identity with the amino acid sequence of SEQ ID NO: 38, or consists of SEQ ID NO: 38;
12) the heavy chain comprises the amino acid sequence as set forth in SEQ ID NO: 43 or an amino acid sequence having at least 90% identity with the amino acid sequence of SEQ ID NO: 43, or consists of SEQ ID NO: 43; and the light chain comprises the amino acid sequence as set forth in SEQ ID NO: 39 or an amino acid sequence having at least 90% identity with the amino acid sequence of SEQ ID NO: 39, or consists of SEQ ID NO: 39;
13) the heavy chain comprises the amino acid sequence as set forth in SEQ ID NO: 43 or an amino acid sequence having at least 90% identity with the amino acid sequence of SEQ ID NO: 43, or consists of SEQ ID NO: 43; and the light chain comprises the amino acid sequence as set forth in SEQ ID NO: 40 or an amino acid sequence having at least 90% identity with the amino acid sequence of SEQ ID NO: 40, or consists of SEQ ID NO: 40;
14) the heavy chain comprises the amino acid sequence as set forth in SEQ ID NO: 43 or an amino acid sequence having at least 90% identity with the amino acid sequence of SEQ ID NO: 43, or consists of SEQ ID NO: 43; and the light chain comprises the amino acid sequence as set forth in SEQ ID NO: 41 or an amino acid sequence having at least 90% identity with the amino acid sequence of SEQ ID NO: 41, or consists of SEQ ID NO: 41.

5. The antibody or antigen-binding fragment thereof that binds to BDCA-2 of any one of claims 1-4, wherein the antigen-binding fragment is Fab, Fab'-SH, Fv, scFv, or (Fab')₂ fragment.

6. An isolated polynucleotide molecule encoding the antibody or antigen-binding fragment thereof of any one of claims 1-5.

7. A vector comprising the polynucleotide molecule of claim 6.

8. A host cell comprising the vector of claim 7 or the polynucleotide molecule of claim 6.

9. A conjugate comprising the antibody or antigen-binding fragment thereof of any one of claims 1-5 and a conjugating moiety that is another molecule; preferably, the conjugating moiety is a radioisotope, a fluorescent substance, a luminescent substance, a colored substance, or an enzyme.

10. A pharmaceutical composition comprising the antibody or antigen-binding fragment thereof of any one of claims 1-5 or the polynucleotide molecule of claim 6 or the vector of claim 7 or the host cell of claim 8 or the conjugate of claim 9 and a pharmaceutically acceptable carrier.

11. A method of preparing an antibody or antigen-binding fragment thereof that binds to BDCA-2, the method including culturing a host cell comprising a polynucleotide molecule encoding the antibody or antigen-binding fragment thereof of any one of claims 1-5, under conditions suitable for expression of the antibody, optionally the method further including recovering the antibody or antigen-binding fragment thereof from the host cell.

12. Use of the antibody or antigen-binding fragment thereof of any one of claims 1-5, or the polynucleotide molecule of claim 6, or the vector of claim 7, or the host cell of claim 8, or the conjugate of claim 9, or the composition of claim 10, in the preparation of a medicament for treating a disease in a subject.

13. The use of claim 12, wherein the disease is an autoimmune disease or cancer, such as autoimmune diseases like Systemic Lupus Erythematosus (SLE), scleroderma, polymyositis, dermatomyositis, psoriasis, Sjogren's syndrome, rheumatoid arthritis, Graves' disease and Hashimoto's disease; breast tumor, ovarian cancer, Multiple Myeloma (MM), and the like.

14. A method of reducing, eliminating pDC cells in circulation, including administering to the subject an effective amount of the antibody or antigen-binding fragment thereof of any one of claims 1-5 or the polynucleotide molecule of claim 6 or the vector of claim 7 or the host cell of claim 8 or the conjugate of claim 9 or the pharmaceutical composition of claim 10.

15. A method of preventing and/or treating a tumor or an autoimmune disease in a subject, the method including administering to the subject in need thereof an effective amount of the antibody or antigen-binding fragment thereof of any one of claims 1-5 or the polynucleotide molecule of claim 6 or the vector of claim 7 or the host cell of claim 8 or the conjugate of claim 9 or the pharmaceutical composition of claim 10.

16. A kit comprising the antibody or antigen-binding fragment thereof of any one of claims 1-5 or the conjugate of claim 9.

17. A method of detecting the presence or level of BDCA-2 in a sample including the steps of contacting the antibody or antigen-binding fragment thereof of any one of claims 1-5 or the conjugate of claim 9 with the sample and detecting whether the antibody or antigen-binding fragment thereof or conjugate forms a complex with BDCA-2.
